# EUROPEAN PATENT SPECIFICATION

(11) **EP 4 362 916 B1**
(45) Date of publication and mention of the grant of the patent: **16.09.2026**
(21) Application number: 23727538.3
(22) Date of filing: 17.05.2023
(51) Int. Cl.: A61K 9/06, A61K 31/00

(54) **AQUEOUS GEL COMPOSITION**
WÄSSRIGE GELZUSAMMENSETZUNG
COMPOSITION DE GEL AQUEUX

(30) Priority: 18.05.2022 EP 22174123
(43) Date of publication of application: 08.05.2024
(73) Proprietor: Acousia Therapeutics GmbH, 72070 Tübingen (DE)
(72) Inventor: HEYMANS, Sven, 3660 Oudsbergen (BE); KOOL, Peter Jan Robert, 6074 AG Melick (NL); DIEDERICHS, Julia Eva, 20129 Milano (IT); DYHRFJELD-JOHNSEN, Jonas, 72127 Kusterdingen (DE)
(74) Representative: Patentanwälte Ruff, Wilhelm, Beier, Dauster & Partner mbB
(86) International application number: PCT/EP2023/063320
(87) International publication number: WO 2023/222795

(56) References cited:
- EP-A1- 3 567 034
- US-A1- 2002 076 422
- US-A1- 2017 216 439
- US-A1- 2018 228 903

## Description

### TECHNICAL FIELD AND PRIOR ART

The present invention relates to a novel aqueous gel composition, in particular useful as a carrier of pharmaceutically active agents. The present invention also relates to a pharmaceutical composition or medicament comprising this aqueous gel composition, in particular with a sustained release of pharmaceutical active agents, and the use of this aqueous gel composition in the prevention or treatment of inner ear diseases.

Because of their viscosity properties gel compositions are often used as carriers for topical and oral administration of pharmaceutically active agents. Important for the administration of gel compositions are e.g. specific viscosity properties that on the one hand allow an easy application by a syringe, in particular a sufficient syringeability, i.e. the capability of being dispensed from, and/or drawn into, a syringe, or an easy application by a spatula and on the other hand a good solubilization of the pharmaceutically active agent which allows the gel composition to serve as a depot with a sustained release of the pharmaceutical active agent and a good adhesion of the composition at organic tissues at about body temperature, in particular for a period of at least 3 days, preferably of at least 5 days, after a single administration at organic tissue.

Therefore, thermoreversible non-aqueous gel compositions, whose viscosity changes at a characteristic temperature, comprising mainly a particulate gel matrix, with viscosity increasing thickeners in an amount sufficient to provide a gelation temperature at about body temperature, are often used.

However, despite being a good carrier of pharmaceutically active agents that have relatively poor water solubility properties non-aqueous particulate gel compositions show the disadvantage of not being able to maintain the viscosity properties and a homogenous distribution of particles over a long period of time, due to precipitation of the particles. In particular, gel compositions containing an oily matrix and thickeners (e.g. oleogels), which remain undissolved, show this disadvantage. Further, precipitation of thickening agents results in an in-homogeneously distributed pharmaceutically active agent and entails the risk of inconsistent release of the pharmaceutical active agent. Therefore, a subsequent pre-treatment, in particular involving heating and sonication, are often used to establish a re-homogenization of the pharmaceutically active agent immediately before application. However, this pre-treatment is difficult to implement in daily clinical practice and is therefore undesirable.

Aqueous gel compositions offer a good alternative as gels with viscosity properties that would allow a persistent good syringeability and good adhesion at organic tissues. Further, because of the uniform distribution and good solubility of most pharmaceutically active agents in aqueous gel compositions, aqueous gel compositions allow a constant sustained release of pharmaceutically active agents.

However, for pharmaceutically active agents that are having relatively poor water solubility properties conventional aqueous gel compositions lack those properties and are not suitable carriers.

Especially for the administration of gel compositions at internal organs, there is a need to administer pharmaceutical compositions with pharmaceutically active agents that are having relatively poor water solubility properties with a syringe. Further, internal organs such as eyes, ears, nose, mouth, lip, vagina, urethral opening and anus are mostly covered by mucus or mucous membranes which makes adhesion (mucoadhesion) challenging.

Therefore, conventional aqueous gel compositions cannot facilitate both a proper viscosity, which would allow a good syringeability as well as a good adhesion at organic tissues and a good administration as well as a sustained release of pharmaceutically active agents that are having relatively poor water solubility properties.

Thermoreversible aqueous gel compositions are known from EP 3501521 A1 and WO 2019210107. The corresponding compositions comprise pharmaceutically active agents like cisplatin, carboplatin, oxaliplatin or corticosteroids, in particular Dexamethason or JNK (c-Jun N-terminal kinase) inhibitors, for the treatment or prevention of drug induced ototoxicity.

US 20180000950 A1 describes a non-aqueous gel composition comprising a therapeutic agent, triglycerides and at least one viscosity modulating agent. The triglycerides are present in an amount that is sufficient to stabilize the therapeutic agent for injection into the ear. Preferably the at least one viscosity modulating agent is silicon dioxide.

There are some patent documents comprising non-aqueous thermoreversible gels for the use in the treatment of different diseases, for example WO 2014076569 A2 and EP 0530965 A1.

EP 0530965 A1 describes a composition for nasal application comprising at least one sexual hormonic drug, at least one lipophilic carrier and a surfactant. Further, the composition comprises a viscosity regulating agent which is colloidal silicon dioxide.

US 2013150410 A1 describes a method of treating otic disorders selected from a group consisting of Meniere's Disease, endolymphatic hydrops, progressive hearing loss, dizziness, Vertigo and tinnitus, comprising transtympanic administration of a sterile pharmaceutical composition into the ear. The composition comprises a multiparticulate ion channel modulating agent such that sustained release of the ion channel modulating agent into the ear occurs for a period of at least 5 days.

US 2019038469 A1 claims a system for delivering a therapeutic composition to the inner ear. Further a system comprising a delivery sheath having a lumen and a cannula configured to be inserted through the lumen of the delivery sheath is described. The cannula has a lumen for delivery of the therapeutic composition and a distal tip for piercing the tympanic membrane for delivery of the therapeutic composition to the round window membrane so as to have the composition adhere to the round window membrane. Further, the composition comprises a therapeutic agent for treating an inner ear disorder and a thixotropic material which allows the therapeutic composition to exist in a liquid form while being injected through the cannula.

US-2002076422 discloses a cleansing gel composition comprising an alcohol in the form of glycerine, one or more emollients, a first surfactant and a second surfactant and water (see claim 1 and paragraphs [0026] and [0030]).US-2017216439 discloses an aqueous gel composition suitable for local administration to the external auditory canal (EAC). The composition can comprise surfactants, co-surfactants and an otic agent for modulating the production of cerumen. As an otic agent reversible cholinesterase is mentioned (see paragraph [0014]). As surfactants/co-surfactants i.a. Poloxamer 407 and phospholipids are mentioned as one of many possibilities (see paragraph [0062] and [0236]).

It is an object of the present invention to provide a novel gel composition, which has superior properties, compared to conventional gel compositions.

### SUMMARY OF THE INVENTION

The above object and further objects are solved by an aqueous gel composition according to claim 1, a pharmaceutical composition or medicament according to claim 12 and an aqueous gel composition for the use in in the prevention or treatment of inner ear diseases according to claim 13. Preferred embodiments are defined in the dependent claims. Preferably, but not necessarily, said aqueous gel composition is especially suitable for a transtympanic administration and comprises a pharmaceutically active agent with a relatively poor water solubility, as defined in the dependent claims.

The present invention is based in particular on the surprising findings that
- an aqueous gel composition described in the present application is a suitable thermoreversible gel whose sol(liquid)-gel transition temperature can be adjusted in a relatively wide range in contrast to conventional thermoreversible gels by varying the amount and ratio of the at least one first surfactant, the at least one second surfactant and the at least one alcohol,
- an aqueous gel composition described in the present application shows an increased tolerability by patients leading to no or only a few side effects because of the biodegradable compounds of the inventive aqueous gel composition
- all compounds of the aqueous gel composition described in the present application are biodegradable and therefore leave no residues, in particular no non-degradable residues, after administration, in particular after transtympanic administration, which could lead to side effects and thus positively influences a pharmaceutical application,
- an aqueous gel composition described in the present application has an improved stability compared to conventional gel compositions and is therefore able to maintain the viscosity properties and a homogenous distribution of its components, preferably of a pharmaceutically active agent, more preferably a pharmaceutically active agent with low solubility, over a long period of time, and
- an aqueous gel composition described in the present application is easy adjustable concerning its solution of pharmaceutically active agents and therefore its sustained release of pharmaceutically active agents by adaptation of the physicochemical properties of the gel due to selection of different ingredients and ratios thereof.

Preferably, the inventive aqueous gel composition comprises or consists of a hydrophobic portion, preferably comprising the at least one first surfactant and the at least one second surfactant, and a hydrophilic portion, preferably including water and the at least one alcohol.

Preferably, the inventive aqueous gel composition is sterile.

Further, the inventive aqueous gel composition may be a surfactant gel.

The terms used in the claims and in the overall description are defined as follows.

The term , aqueous composition" as used herein, means a composition which comprises water.

The term "surfactant gel" as used herein, means a semi-solid aqueous gel composition having viscoelastic properties, based on surfactants, in particular formed by aggregation of hydrated or solvated surfactant molecules.

The term "surfactant" as used herein, means a compound that lowers the surface tension between two liquids or between a liquid and a solid in a composition, enabling or supporting the formation of a dispersion and improving wettability of the composition. The surfactant can also act as detergent, emulsifier, and foaming agent.

The term "region or structure of the ear" as used herein, means a region of the ear, in particular the middle ear region of the ear, or a structure of the ear, in particular the round window structure of the ear, wherein said structure of the ear is a part of said region of the ear.

The term "transtympanic administration" as used herein, means an injection through the tympanic membrane into the middle ear for administration in the middle ear, in particular on the round window membrane. Wherein said administration on the round window membrane is to facilitate diffusion across the round window membrane.

The term "sustainable release gel" as used herein, means a gel composition comprising at least one pharmaceutically active agent, which provides an equally, sustained and controlled liberation, in particular release, of the at least one pharmaceutically active agent into a body, in particular a human body, and its tissues.

The term "multiparticulate" as used herein, means a plurality of particles of at least one pharmaceutically active agent, wherein the particles are of the same diameter or of different diameter.

The term "micronized" as used herein, means a substance, in particular a pharmaceutically active agent, which is reduced in diameter to particles which are measured in µm.

The term "sterile" as used herein, means free from living germs or microorganisms, in particular aseptic.

The term "mucoadhesion" as used herein, means an adhesion at organic tissues which are mostly covered by mucus or mucous membranes such as eyes, ears, nose, mouth, lip, vagina, urethral opening and anus.

The term "thermoreversible gel" as used herein, means a gel composition, whose viscosity changes at a characteristic temperature. This thermal behavior includes, that the property of the gel is not affected by repeated heating and cooling and that the viscosity of the gel is reversible to its initial state. Thermoreversible gels, in particular thermoreversible aqueous gels contain both hydrophobic and hydrophilic components, and the thermoreversible effect is derived from the delicate balance between the hydrophobic and hydrophilic portions of the composition. Temperature changes the interaction between hydrophilic and hydrophobic segments with water molecules, thus can induce a change in the solubility of the cross-linked network, causing a transition from the sol phase to the gel phase or a transition from the gel phase to the sol phase. Changing the balance of hydrophilicity and hydrophobicity determines the macroscopic dissolved state of cross-linking network in an aqueous solution.

The term "sol phase" as used according to the present invention refers to a flowing fluid while the term "gel phase" as used according to the present invention refers to a non-flowing composition that maintains its integrity.

The term "biodegradable" as used according to the present invention refers to any physical or chemical change in the aqueous gel composition caused by any environmental factor, including light, heat, moisture, wind, chemical conditions or biological activity. Typically, the aqueous gel composition of the present invention is degraded into carbon dioxide, water, and biomass as a result of the action of water and/or living organisms and/or enzymes.

Therefore, most or all compounds of the inventive aqueous gel composition are preferably having the "GRAS" status (Generally Recognized As Safe status established by the FDA (Food and Drug Administration)) and/or are FDA approved and listed in the US and European Pharmacopoeia (European collection of recognized medicinal substances).

According to the invention the viscosity of the inventive aqueous gel composition at a temperature between 9°C and 15°C, in particular of 10°C, is preferably from 100 mPas to 250 mPas, more preferably from 150 mPas to 200 mPas.

In an embodiment of the invention the viscosity of the inventive aqueous gel composition at a temperature of 25°C is from 1400 mPas to 2300 mPas, more preferably from 1600 mPas to 2000 mPas. Within the last-mentioned range viscosities from 1700 mPas to 1800 mPas are even further preferred.

According to the invention the viscosity of the inventive aqueous gel composition at a temperature of 37°C is preferably from 500 to 1200 mPas, more preferably from 700 to 1000 mPas.

Alternatively, the viscosity of the inventive aqueous gel composition at a temperature of 37°C is preferably from 1400 mPas to 2300 mPas, more preferably from 1600 mPas to 2000 mPas. Within the last-mentioned range viscosities from 1700 mPas to 1800 mPas are even further preferred.

Alternatively and/or in combination, the viscosity of the inventive aqueous gel composition at a temperature of 25°C is preferably from 500 to 1200 mPas, more preferably from 700 to 1000 mPas.

Preferably, the viscosity of the inventive aqueous gel composition at a temperature of 10°C is lower than the viscosity at 25°C and the viscosity of the inventive aqueous gel composition at a temperature of 25°C is lower than the viscosity at 37°C, more preferred the viscosity increases from 10°C to 37°C.

Preferably, the inventive aqueous gel composition is gelling at room temperature (from 15°C to 25°C) while being liquid below room temperature (under 15°C), in particular from between 4°C and 15°C, preferably from 5°C to 14°C. Further preferred the inventive aqueous gel composition is gelling at room temperature (from 15°C to 25°C) and stays this way at body temperature (ca. 37°C) while being liquid below room temperature (under 15°C).

In contrast to conventional gel compositions, the inventive aqueous gel composition has the advantage to provide a thermoreversible gel composition, whose viscosity changes at a characteristic temperature.

The thermoreversible viscosity properties mentioned above provide an aqueous gel composition with a viscosity making it especially useful in a pharmaceutical composition or in a medicament for topical administration. A viscosity is provided that is sufficient for a syringeability at (pharmaceutical) room temperature from 15°C to 25°C, i.e. the capability of being dispensed from, and/or drawn into, a syringe, and a viscosity that allows good adhesion of the composition for topical administration at organic tissue at about body temperature normally from 35°C to 38°C, in particular for a period of at least 3 days, preferably of at least 5 days, e.g. after a single administration at organic tissue. In particular the inventive aqueous gel composition is especially useful to be drawn up into the syringe in liquid state, preferably < 10°C, dispensed at room temperature and gelling at body temperature after administration.

This is especially advantageous for the administration of gel compositions at internal organs and/or compartments, where both a need to administer the composition with a syringe, and a need for a good adhesion, especially a good mucoadhesion is given.

The inventive aqueous gel composition preferably comprises a viscosity and gel structure useful to be applied via a suitable needle diameter of 18 to 27 gauge (Birmingham gauge), more preferable of 20 to 23 gauge, in particular of 20 gauge. The inventive aqueous gel composition will be also suitable of being applied in small volumes from 20 µl to 300 µl, preferably 30 µl to 300 µl, more preferably 30 µl to 200 µl, e.g. to the inner ear, in particular for application into the middle ear for diffusion of the pharmaceutically active agent into the inner ear.

The viscosity normally is measured with a viscometer, in particular a rheometer. A rheometer usually is a laboratory device measuring flow characteristics of a liquid, suspension or slurry in response to applied forces. A common device type is a so called shear rheometer, in which a shear stress is applied to the corresponding material. Devices which can be used to measure the viscosity of the aqueous gel compositions according to the invention are rheometers from Brookfield (AMETEK Brookfield).

Preferably, the inventive aqueous gel composition is preferably free of organic macromolecules such as hydroxyethylcellulose, colloidal solid particles, preferably inorganic colloidal solid particles, colloidal silica, ethylene glycol, dioxide, zinc stearate, aluminium stearate and/or organo-modified hectorite (Bentone).

In a further embodiment of the invention the at least one first surfactant comprises or consists of at least one copolymer, in particular at least one block-copolymer.

The at least one first surfactant is a surfactant different from the at least one second surfactant.

The at least one first surfactant is a non-ionic block-copolymer

Further, the at least one first surfactant is an amphiphilic block-copolymer.

The at least one first surfactant is a non-ionic amphiphilic block-copolymer.

The term "copolymer" as used according to the present invention refers to a polymer comprising or consisting of at least two different polymers.

The term "copolymer block" as used according to the present invention refers to a polymer comprising or consisting of at least two different polymer blocks. Preferably the polymer blocks are homopolymer blocks or copolymer blocks. Homopolymer blocks are polymer blocks comprising or consisting of one monomeric compound. If at least two different monomers are linked in one block, the structure is called a block-copolymer.

Copolymers, in particular block-copolymers have the advantage of being able to create an amphiphilic structure with the property of dissolving in both polar and non-polar solvents. They may thus be both hydrophilic and lipophilic. The amphiphilic character of copolymers may lead to self-aggregation of the copolymers into micelles with an inner core constituted by hydrophobic blocks and an outer shell constituted by hydrophilic units.

Examples of block-copolymers comprise or consist of at least two different polymer blocks selected from the group consisting of poly(ethylene oxide) (PEO) block, poly(d, I-lactic acid-co-glycolic acid) (PLGA) block, poly (I-caprolactone) block (PCL), poly(I-lactic acid) (PLA) block, poly(ether ester amide) (PEEA) block, poly(propylene oxide) (PPO) block, poly(N-isoprolylacrylamide) (pNiPAAm) block, poly(ethylene glycol) block (PEG), poly(propylene glycol) (PPG) block, poly(methacrylic acid) (PMAA) block, poly(vinyl alcohol) (PVA) block, poly(vinyl pyrrolidone) (PVP) block and mixtures of at least two of the afore-said polymer blocks.

The at least one block-copolymer comprises or consists of at least one poly(ethylene oxide) block and at least one poly(propylene oxide) block.

The at least one block-copolymer is a poloxamer, in particular poloxamer 407, wherein the last digit (7) multiplied by a factor of 10 indicates approximately the relative mass fraction of the poly(ethylene oxide) blocks in percent, and the preceding digits (40) multiplied by a factor of 100 encode the relative molecular mass of the poly(propylene oxide) block.

Poloxamer 407 is also known as Pluronic F 127, Kolliphor P 407 or Lutrol F 127 and preferably comprises or consists of a central poly(ethylene oxide) block linked to a poly(propylene oxide) block at both ends of the central poly(ethylene oxide) block.

Preferably the central poly(ethylene oxide) block comprises or consists of 15 to 67 poly(ethylene oxide) monomers, preferably 56 poly(ethylene oxide) monomers, and the poly(propylene oxide) blocks linked to both ends of the central poly(ethylene oxide) block each or together comprises or consists of 2 to 130 poly(ethylene oxide) monomers, preferably 101 poly(ethylene oxide) monomers. Preferably the number of monomers in each poly(ethylene oxide) block can be different or the same.

Poloxamer 407 has the advantage of being an amphiphilic structure with the property of dissolving in both polar and non-polar solvents, thus being both hydrophilic and lipophilic.

In the invention the at least one second surfactant comprises or consists of at least one phospholipid.

The at least one second surfactant is a surfactant different from the at least one first surfactant.

Further, the at least one first surfactant and the at least one second surfactant are both amphiphilic surfactants.

The at least one first surfactant and the at least one second surfactant comprises or consists of surfactants selected from the group consisting of ionic, non-ionic and zwitterionic surfactants.

Further, the at least one second surfactant is an amphiphilic phosphatidylcholine.

Further, the at least one second surfactant is a zwitterionic phosphatidylcholine.

The at least one second surfactant is a zwitterionic amphiphilic phosphatidylcholine.

Preferably, the phospholipids comprise or consist of a glycerol backbone or an amino-alcohol sphingosine backbone, which is esterified to at least one, preferably two, fatty acids, a phosphate group and a hydrophilic residue. The phospholipids with two esterified fatty acids are called diacylphospholipids, whereas phospholipids with one fatty acid are called monoacyl-phospholipids or lyso-phospholipids.

In a further embodiment of the invention the at least one phospholipid is selected from the group consisting of phosphatidylcholine, cholesterol, phosphatidylethanolamine, phosphatidylserine, phosphatidylglycerol, phosphatidylinositol and mixtures of at least two of the afore-said phospholipids.

Preferably, the at least one phospholipid is at least one phosphatidylcholine.

Further, the phosphatidylcholine preferably is a diacyl-phosphatidylcholine or monoacylphosphatidylcholine, more preferably a natural phosphatidylcholine or synthetic phosphatidylcholine, in particular a natural diacyl-phosphatidylcholine or a natural monoacylphosphatidylcholine or a synthetic diacyl-phosphatidylcholine or a synthetic monoacylphosphatidylcholine.

The term "natural phosphatidylcholine" as used herein, means a phosphatidylcholine derived from plants, in particular from soy plants.

While phosphatidylserine, phosphatidylglycerol and phosphatidylinositol are negatively charged at physiological pH, and phosphatidylethanolamine is negatively charged at basic pH and zwitterionic at physiological pH, phosphatidylcholine has the advantage of being zwitterionic and amphiphilic at physiological pH, with the property of dissolving in both polar and non-polar solvents. Thus, phosphatidylcholine is both hydrophilic and lipophilic.

In a further embodiment of the invention the at least one alcohol comprises or consists of at least one polyol, in particular at least one diol.

The most important common advantageous feature of diol, in particular alkanol, is hydrophilicity. This property decreases with increasing length of the alkyl radical and increases with the number of hydroxyl groups. Diols have a higher hydrophilicity than monohydric (one hydroxyl group) alcohols, in particular monohydric alkanols. Especially the short-chain diols, in particular alkanols, such as propylene glycol, are advantageous, in particular as solvent, because of its amphiphilic character.

The at least one diol is at least one propylene glycol.

Further, propylene glycol is miscible with water and amphiphilic, which is in particular advantageous in combination with an amphiphilic at least one first surfactant and an amphiphilic at least one second surfactant, resulting in optimal interaction with the at least one first surfactant and the at least one second surfactant in the inventive aqueous gel composition and therefore contributing to the gel structure.

Preferably, the at least one alcohol and the at least one second surfactant are in the form of a premixed composition. Useful premixed compositions are, for example, commercially available under the name Phosal^{®}, preferably Phosal^{®} 50PG. Phosal^{®} 50PG comprises or consist of phosphatidylcholine, in particular of phosphatidylcholine derived from soy plants, and propylene glycol, preferably in a mass ratio of 1 : 1.

In a further embodiment of the invention the at least one first surfactant is present in an amount from 10 % to 25 % by weight, preferably from 12 % to 22 % by weight, more preferably from 14 % to 16 % by weight, based on the total weight of the composition.

The amount of the at least one first surfactant in the aqueous gel composition has been found important to ensure formation of a thermoreversible gel with the required transition temperature from sol phase to gel phase, good carriage of the pharmaceutical active agent in the inventive aqueous gel composition, in particular good encapsulation capacity of the pharmaceutical active agent in the inventive aqueous gel composition, mucoadhesive properties and a sustained release of the pharmaceutical active agent from the inventive aqueous gel composition, in particular good sustained release kinetics.

In a further embodiment of the invention the at least one second surfactant is present in an amount from 6 % to 20 % by weight, preferably from 6 % to 15 % by weight, more preferably from 6 % to 10 % by weight, based on the total weight of the composition.

In a further embodiment of the invention the at least one alcohol is present in an amount from 6 % to 20 % by weight, preferably from 6 % to 15 % by weight, more preferably from 6 % to 10 % by weight, based on the total weight of the composition.

Preferably the at least one alcohol and the at least one second surfactant is present in the composition in a mass ratio of 1 : 1, 30 : 70 or 70 : 30.

The amount of the at least one second surfactant and the at least one alcohol in the aqueous gel composition has been found to be especially advantageous to ensure formation of a thermoreversible gel with the required transition temperature from sol phase to gel phase, good carriage of the pharmaceutical active agent in the inventive aqueous gel composition, in particular good encapsulation capacity of the pharmaceutical active agent in the inventive aqueous gel composition, mucoadhesive properties and a sustained release of the pharmaceutical active agent from the inventive aqueous gel composition, in particular good sustained release kinetics.

According to the invention an aqueous gel composition is disclosed, wherein:
- the at least one first surfactant is a poloxamer, in particular being present in an amount from 14 % to 16 % by weight, based on the total weight of the composition,
- the at least one second surfactant is a phospholipid, preferably a natural phosphatidylcholine, in particular being present in an amount from 6 % to 10 % by weight, based on the total weight of the composition, and
- the at least one alcohol is propylene glycol, in particular being present in an amount from 6 % to 10 % by weight, based on the total weight of the composition.

It was found that the inventive aqueous gel composition is especially useful for providing an in vivo sustained release of a therapeutically effective amount of pharmaceutically active agents after administration, preferably at internal organs and/or compartments, preferably for application into the middle ear for diffusion of the pharmaceutically active agent into the inner ear, in particular after transtympanic administration, for a period of at least 3 days, preferably at least 5 days, after a single administration at organic tissue.

According to the invention the aqueous gel composition further comprises at least one pharmaceutically active agent.

Preferably, the inventive aqueous gel composition is a sustainable release gel, which comprises at least one pharmaceutically active agent, which provides an equally, sustained and controlled liberation, in particular release, of the at least one pharmaceutically active agent into a body, in particular a human body, over a period of at least 3 days, preferably of at least 5 days, more preferably of at least 1 month after a single administration, in particular at organic tissue.

The at least one pharmaceutically active agent is preferably present in an amount from 0,05 % to 10 % by weight, preferably from 0,1 % to 10 % by weight, more preferably from 2 % to 10 % by weight, further preferred 2 % to 5 % by weight, based on the total weight of the composition.

The amount of the at least one pharmaceutically active agent as described above, was found to be especially useful for providing a sustainable release gel, releasing the pharmaceutically active agent from the inventive aqueous gel composition, for a period of at least 3 days, preferably at least 5 days, after a single administration at organic tissue.

Further, the at least one pharmaceutically active agent may in particular be in the form of particles and has a mean particle diameter from 0,01 µm to 100 µm, preferably from 5 µm to 80 µm, more preferably from 20 µm to 50 µm.

The term "mean particle diameter" as used herein, means a particle or 50% of all particles of the at least one pharmaceutically active agent (D50) whose dimensions, in particular its diameter, preferably mean diameter, lies in a range from 0,01 µm to 100 µm, preferably from 5 µm to 80 µm, more preferably from 20 µm to 50 µm. In this context, the term "diameter" is to be understood in the case of a spherical particle to mean the diameter of the sphere, i.e. twice the radius of the sphere. In the case of a non-spherical particle, the term "diameter" is to be understood as the largest possible distance that two points along a circumference of the particle can take from each other.

More preferably, the at least one pharmaceutically active agent is in the form of particles, wherein 90% of all particles (D90) of the at least one pharmaceutically active agent are smaller than a particle diameter, in particular mean particle diameter, of 0,01 µm to 100 µm, preferably of 1 µm to 50 µm, more preferably from 5 µm to 20 µm, even more preferred 11 µm or 7 µm.

Further preferred, the at least one pharmaceutically active agent is in the form of particles, wherein 50% of all particles (D50) of the at least one pharmaceutically active agent are smaller than a particle diameter, in particular mean particle diameter, of 0,01 µm to 100 µm, preferably of 1 µm to 50 µm, more preferably from 5 µm to 10 µm, even more preferred 5 µm.

The mean particle diameter distribution is preferably obtained by laser diffraction. During a laser diffraction measurement, a laser beam passes through a dispersed particulate sample and the angular variation in intensity of the scattered light is measured. Large particles scatter light at small angles relative to the laser beam and small particles scatter light at large angles. The angular scattering intensity data is then analyzed to calculate the diameter of the particles that created the scattering pattern using the Mie theory of light scattering. The particle diameter is reported as a volume equivalent sphere diameter. The folded optical design in the Mastersizer 3000 (from Malvern Panalytical), which was in particular used for the measurements provides a particle diameter range from 10 nm up to 3.5 mm using a single optical measurement path. The Mastersizer 3000 uses a sequential combination of measurements with red and blue light sources to measure across the entire particle diameter range.

The above-described mean particle diameter of the at least one pharmaceutically active agent was found to be especially useful to obtain a homogenous distribution of the at least one pharmaceutically active agent in the aqueous gel composition.

Preferably the desired particle diameter of the at least on pharmaceutically active agent has been obtained by grinding, ball milling, high pressure homogenization, homogenization, micronization or a combination of at least two of the described methods.

Further, due to the above-described particle diameter of the at least one pharmaceutically active agent the uptake of the at least one pharmaceutically active agent from the inventive composition into a body, after administration at organic tissues, is especially advantageous in comparison to other conventional gel compositions comprising pharmaceutically active agents. Due to the advantageous uptake of the pharmaceutically active agent from the inventive aqueous gel composition into the body, lesser amounts of pharmaceutically active agent in the composition are needed to achieve the same therapeutically effect as conventional gel compositions.

Alternatively, the at least one pharmaceutically active agent may in particular not be micronized.

The term "uptake of a pharmaceutically active agent into a body" as used herein, means an uptake of a pharmaceutically active agent into a mammal body, in particular a human and/or an animal body, in particular into the perilymph and/or endolymph of a mammal body, in particular of a human and/or of an animal body.

Preferably, the inventive aqueous gel composition comprises, based on the concentration of the pharmaceutically active agent, a multiparticulate suspension of the pharmaceutically active agent.

In a preferred embodiment of the invention the inventive aqueous gel composition preferably comprises a solution of the pharmaceutically active agent.

Preferably the at least one pharmaceutically active agent has a (low) solubility in water at room temperature (22-26°C) of < 1 mg/ml, preferably < 0,1 mg/ml, more preferably < 0,01 mg/ml, in particular from 0,01 mg/ml to 1 mg/ml.

The low solubility of pharmaceutically active agents negatively affects the uptake of the pharmaceutically active agent into the body, in particular in the perilymph and/or endolymph of the body, after administration, in particular at internal organs, e.g. after transtympanic administration. As a consequence, a therapeutic effect cannot be achieved by a single administration, but a local depot formulation has to be developed which uses the intrinsic low solubility of the pharmaceutically active agents in a suitable gel composition to obtain a sustained uptake of the pharmaceutically active agents over the desired time interval.

The inventive aqueous gel composition shows the advantage of being especially useful for carrying pharmaceutically active agents, having a (low) solubility as described above, in particular for a topical administration.

Preferably, at least one first surfactant, in particular poloxamer, and the at least one second surfactant, in particular phospholipid, are both amphiphilic compounds which act as surfactants and form lamellar structures together in the inventive aqueous gel composition. Due to the different carbon chain lengths of the at least one first surfactant, in particular poloxamer, and the at least one second surfactant, in particular phospholipids, cavities are preferably generated in the lamellar structure where the drug substance can be intercalated. Therefore, the pharmaceutical active agent is preferably encapsulated in the inventive aqueous gel composition. This may increase the solubilising effect of the formulation and increase the drug load of pharmaceutical active agents, in particular pharmaceutical active agents with low water solubility properties and allows the solubilisation of even poorly water soluble pharmaceutical active agents in the inventive aqueous gel composition.

The term "lamellar structure" as used herein, means a microstructure that comprises alternating layers of different materials, in particular the at least one first surfactant, preferably poloxamer, and the at least one second surfactant, preferably phospholipid.

Further, the inventive aqueous gel composition can be administered to form a local depot to obtain a sustained uptake of the pharmaceutically active agents for a period of at least 3 days, preferably at least 5 days, in particular after a single administration at organic tissue.

Preferably the at least one pharmaceutically active agent is a substance comprising low solubility properties and high permeability properties.

The invention comprises at least one pharmaceutically active agent which may have the formula I: 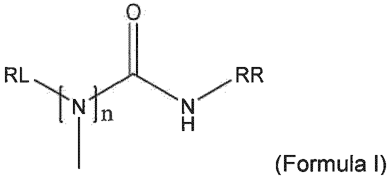 wherein
- n = 1,
- RL is a substituent selected from the group consisting of substituted cyclopentathienyl groups, wherein said substituted cyclopentathienyl groups are substituted with at least one F-atom or Cl-atom, and of unsubstituted or substituted indanyl groups, wherein said substituted indanyl groups are substituted with at least one F-atom or Cl-atom, and
- RR is a substituted phenyl group, wherein said substituted phenyl group consists of at least one substituent selected from F, SF₅, CF₃, and OCF₃.

In a further embodiment of the invention the at least one pharmaceutically active agent is selected from the group consisting of
(S)-3-(3,4-difluorophenyl)-1-(2-chloro-5,6-dihydro-4H-cyclopenta[b]thiophen-6-yl)-1-methylurea,
(S)-3-(3-pentafluorosulfanylphenyl)-1-(2-chloro-5,6-dihydro-4H-cyclopenta[b]thiophen-6-yl)-1-methylurea,
(S)-3-(3,4-difluorophenyl)-1-(2,3-dihydro-1H-inden-1 yl)-1-methylurea,
(S)-3-(3,4-difluorophenyl)-1-(5-chloro-2,3-dihydro-1H-inden-1 yl)-1-methylurea,
(S)-3-(3,4-difluorophenyl)-1-(5-fluoro-2,3-dihydro-1H-inden-1yl)-1-methylurea, and
(S)-1-(5-chloro-2,3-dihydro-1-H-inden-1-yl)-1-methyl-3-(3-pentafluorosulfanylphenyl) urea,
and mixtures of at least two of the afore-said pharmaceutically active agents.

Preferably the the at least one pharmaceutically active agent is (S)-3-(3,4-difluorophenyl)-1-(5-chloro-2,3-dihydro-1H-inden-1yl)-1-methylurea.

The pharmaceutically active agents listed above belong to a class of compounds which preferably function as potassium channel openers, in particular as openers of the Kv7.4 potassium channel and are therefore especially useful in the treatment of disorders associated with an aberrant potassium activity like Alzheimer's disease and Parkinson's disease. Further disorders are neurologic conditions such as epilepsy or cognitive psychiatric disorders like depression, mania and schizophrenia. In particular, it is known that potassium channels play an important role for the normal function of the outer hair cells (OHC) in the organ of the Corti of the cochlea. Therefore, the pharmaceutically active agents listed above are promising candidates for the treatment and/or prophylaxis of hearing loss, e.g. before a treatment with an ototoxicity inducing drug.

In a further embodiment of the invention the at least one pharmaceutically active agent preferably has the formula II: 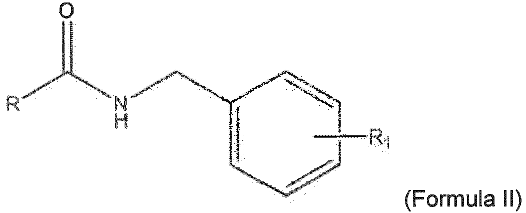 wherein
- R is a unsubstituted cycloalkyl group, in particular bicycloalkyl group, an unsubstituted or substituted phenyl group, or an unsubstituted or substituted thienyl group, wherein said substituted thienyl group or phenyl group is substituted with at least one halogen, preferably selected from a group consisting of at least one F- Atom, Cl- Atom, Br- Atom or I-Atom, more preferably at least one F-atom or at least one Cl-atom, and
- R₁ is F, SF₅, CF₃ or OCF₃.

According to the invention the at least one pharmaceutically active agent is preferably selected from a group consisting of
(1R,2R,4S)-rel-N-(3-(pentafluorosulfanyl)benzyl)bicyclo[2.2.1]heptane-2-carboxamide,
(1S,2S,4R)-N-(3-(pentafluorosulfanyl)benzyl)bicyclo[2.2.1]heptane-2-carboxamide, or stereoisomers or tautomers of
(1S,2S,4R)-N-(3-(pentafluorosulfanyl)benzyl)bicyclo[2.2.1]heptane-2-carboxamide in particular
the enantiomer (1S,2S,4R)-N-(3-(pentafluoro-λ6-sulfaneyl)benzyl)bicyclo[2.2.1]heptane-2-carboxamide or
the racemic mixture (1SR,2SR,4RS)-N-(3-(pentafluoro-λ6-sulfaneyl)benzyl)bicyclo[2.2.1] heptane-2-carboxamide),
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide,
(1S,2S,4R)-N-(3-(trifluoromethoxy)benzyl)bicyclo[2.2.1]heptane-2-carboxamide,
p-Chloro-N-(4-trifluoromethoxy)benzyl)benzamide,
p-Chloro-N-(4-(pentafluorosulfanyl)benzyl)benzamide,
p-Fluoro-N-(4-trifluoromethoxy)benzyl)benzamide,
p-Fluoro-N-(4-(pentafluorosulfanyl)benzyl)benzamide,
p-Chloro-N-(4-(trifluoromethyl)benzyl)benzamide,
p-Fluoro-N-(4-(trifluoromethyl)benzyl)benzamide
and mixtures of at least two of the afore-said pharmaceutically active agents.

Preferably the the at least one pharmaceutically active agent is
(1R,2R,4S)-rel-N-(3-(pentafluorosulfanyl)benzyl)bicyclo[2.2.1]heptane-2-carboxamide
or (1S,2S,4R)-N-(3-(pentafluorosulfanyl)benzyl)bicyclo[2.2.1]heptane-2-carboxamide,

The pharmaceutically active agents listed above belong to a class of compounds which preferably function as potassium channel openers, in particular as openers of the Kv7.4 potassium channel and are therefore especially useful in the treatment of disorders associated with an aberrant potassium activity like Alzheimer's disease and Parkinson's disease. Further disorders are neurologic conditions such as epilepsy or cognitive psychiatric disorders like depression, mania and schizophrenia. In particular, it is known that potassium channels play an important role for the normal function of the outer hair cells (OHC) in the organ of the Corti of the cochlea. Therefore, the pharmaceutically active agents listed above are promising candidates for the treatment and/or prophylaxis of hearing loss, e.g. before a treatment with an ototoxicity inducing drug.

The pharmaceutically active agent described above, in particular (1SR,2SR,4RS)-N-(3-(pentafluoro-λ6-sulfanyl)benzyl)bicyclo[2.2.1]heptane-2-carboxamide (in the following called Compound A), the enantiomer (1S,2S,4R)-N-(3-(pentafluoro-λ6-sulfaneyl)benzyl)bicyclo[2.2.1]heptane-2-carboxamide thereof (in the following called Compound B), show a (low) solubility in water at room temperature (22-26°C) of < 1 mg/ml, preferably < 0,1 mg/ml, more preferably < 0,01 mg/ml, in particular from 0,01 mg/ml to 1 mg/ml.

Further, an aqueous gel composition is preferred, wherein:
- the at least one first surfactant is a poloxamer, in particular being present in an amount from 14 % to 16 % by weight, based on the total weight of the composition,
- the at least one second surfactant is a phospholipid, preferably a natural phosphatidylcholine, in particular being present in an amount from 6 % to 10 % by weight, based on the total weight of the composition, and
- the at least one alcohol is propylene glycol, in particular being present in an amount from 6 % to 10 % by weight, based on the total weight of the composition,
- in particular the viscosity of the composition at a temperature of 25°C or 37°C is from 1400 to 2300 mPas, preferably from 1600 to 2000 mPas and
- the at least one pharmaceutically active agent is selected from a group consisting of
   (S)-3-(3,4-difluorophenyl)-1-(2-chloro-5,6-dihydro-4H-cyclopenta[b]thiophen-6-yl)-1-methylurea,
   (S)-3-(3-pentafluorosulfanylphenyl)-1-(2-chloro-5,6-dihydro-4H-cyclopenta[b]thiophen-6-yl)-1-methylurea,
   (S)-3-(3,4-difluorophenyl)-1-(2,3-dihydro-1 H-inden-1 yl)-1-methylurea,
   (S)-3-(3,4-difluorophenyl)-1-(5-chloro-2,3-dihydro-1H-inden-1yl)-1-methylurea,
   (S)-3-(3,4-difluorophenyl)-1-(5-fluoro-2,3-dihydro-1H-inden-1yl)-1-methylurea, and
   (S)-1-(5-chloro-2,3-dihydro-1-H-inden-1-yl)-1-methyl-3-(3-pentafluorosulfanylphenyl) urea,
   (1R,2R,4S)-rel-N-(3-(pentafluoro-λ6-sulfanyl)benzyl)bicyclo[2.2.1]heptane-2-carboxamide,
   (1S,2S,4R)-N-(3-(pentafluoro-λ6-sulfanyl)benzyl)bicyclo[2.2.1]heptane-2-carboxamide, or stereoisomers or tautomers of
   (1S,2S,4R)-N-(3-(pentafluoro-λ6-sulfanyl)benzyl)bicyclo[2.2.1]heptane-2-carboxamide in particular
   the enantiomer (1S,2S,4R)-N-(3-(pentafluoro-λ6-sulfaneyl)benzyl)bicyclo[2.2.1] heptane-2-carboxamide or
   the racemic mixture (1SR,2S,4R)-N-(3-(pentafluoro-λ6-sulfaneyl)benzyl) bicyclo[2.2.1] heptane-2-carboxamide),
   (1R,2R,4S)-rel-N-(3-(trifluormethyl)benzyl)bicyclo[2.2.1]heptane-2-carboxamide,
   (1R,2R,4S)-rel-N-(3-(trifluoromethoxy)benzyl)bicyclo[2.2.1]heptane-2-carboxamide,
   (1S,2S,4R)-N-(3-(trifluoromethyl)benzyl)bicyclo[2.2.1]heptane-2-carboxamide,
   (1S,2S,4R)-N-(3-(trifluoromethoxy)benzyl)bicyclo[2.2.1]heptane-2-carboxamide,
   p-Chloro-N-(4-trifluoromethoxy)benzyl)benzamide,
   p-Chloro-N-(4-(pentafluorosulfanyl)benzyl)benzamide,
   p-Fluoro-N-(4-trifluoromethoxy)benzyl)benzamide,
   p-Fluoro-N-(4-(pentafluorosulfanyl)benzyl)benzamide,
   p-Chloro-N-(4-(trifluoromethyl)benzyl)benzamide,
   p-Fluoro-N-(4-(trifluoromethyl)benzyl)benzamide,
   and mixtures of at least two of the afore-said pharmaceutically active agents.

The composition mentioned above was found to be especially advantageous, concerning the adhesion and viscosity properties and the uptake of a pharmaceutically active agent from the inventive aqueous gel composition into the body providing a sustained release of the compound for a period of at least 3 days, preferably at least 5 days, after a single administration at organic tissue.

According to a second aspect, the present invention relates to a container, in particular a discharging device, wherein the container contains, preferably is filled with, an aqueous gel composition, in particular with an aqueous gel composition according to the first aspect of the invention.

The container may be in the form of a vial or syringe.

Preferably, the discharging device is connected to a suitable needle for transtympanic administration, preferably a needle with a diameter of 18 to 27 gauge (Birmingham gauge), more preferable of 20 to 23 gauge, in particular of 20 gauge, suitable to apply small volumes from 20 µl to 200 µl of the inventive aqueous gel composition, in particular for transtympanic administration.

Further, the discharging device is preferably a CycloOlefinPolymer (COP) syringe, which shows the advantage of bearing no risk to have any residual glass particles from the manufacturing process included.

Preferably, the discharging device comprises a stopper and a plunger.

Further, the discharging device preferably comprises a syringe holder, in particular a plastic or cardboard syringe holder, which is especially useful to avoid any damage of the syringe during transport.

Further, the discharging device, in particular the syringe, is preferably filled under aseptic conditions with a sterile, inventive aqueous gel composition.

With respect to further features and advantages of the discharging device, in particular in terms of the aqueous gel composition, reference is made in its entirety to the features and advantages described in terms of the aqueous gel composition according to the first aspect of the invention. The features and advantages described under the first aspect of the invention do apply, mutatis mutandis, with respect to the discharging device according to the second aspect of the invention.

According to a third aspect, the invention relates to a pharmaceutical composition or medicament comprising an aqueous gel composition, in particular comprising an aqueous gel composition according to the first aspect of the invention.

With respect to further features and advantages of the pharmaceutical composition or a medicament, in particular in terms of the aqueous gel composition, reference is made in its entirety to the features and advantages described in terms of the aqueous gel composition according to the first aspect of the invention and in terms of the use according to the fourth aspect of the invention. The features and advantages described under the first, second and fourth aspect of the invention do apply, mutatis mutandis, with respect to the pharmaceutical composition or medicament according to the fourth aspect of the invention.

According to a fourth aspect, the invention relates to an aqueous gel composition, in particular an aqueous gel composition according to the first aspect of the invention, for use in the prevention or treatment of inner ear diseases or to a pharmaceutical composition or a medicament for use in the prevention or treatment of inner ear diseases. Preferably said aqueous gel composition or said pharmaceutical composition or said medicament is provided for transtympanic administration.

Preferably, the aqueous gel composition comprising at least one pharmaceutically active agent is administered via transtympanic administration providing a sustained release of the pharmaceutically active agent into the ear, preferably the inner ear, for a period of at least 3 days, preferably at least 5 days, in particular after a single administration. Because of its viscosity properties described above the inventive aqueous gel composition is especially useful for a sustained release of a pharmaceutically active agent, especially for transtympanic administration for the treatment of otic disorders including but not limited to induced hearing loss, in particular sensorineural hearing loss, and drug-induced ototoxicity.

Further, the inventive aqueous gel composition advantageously shows a well-regulated viscosity and gel structure especially suitable for administration as droplets. Following transtympanic administration, the composition preferably adheres, in particular in form of a droplet, preferably droplet comprising the inventive aqueous gel composition in volumes from 20 µl to 300 µl, preferably 30 µl to 300 µl, more preferably 30 µl to 200 µl, on the round window membrane or adjuvant mucosa and is not washed away from the middle ear and/or the round window membrane and is therefore providing a sustained release of the pharmaceutically active agent for a period of at least 3 days, preferably at least 5 days, after a single administration at organic tissue, thereby avoiding frequent transtympanic administration into a patients ear.

With respect to further features and advantages of the use in a pharmaceutical composition or a medicament, in particular in terms of the aqueous gel composition, reference is made in its entirety to the features and advantages described in terms of the aqueous gel composition according to the first aspect of the invention. The features and advantages described under the first and second aspect of the invention do apply, mutatis mutandis, with respect to the use according to the fourth aspect of the invention.

Further features and advantages of the invention will become clear from the following description of preferred embodiments in form of examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### EXAMPLE SECTION

The figures show the following:
Fig. 1a-d: Temperature-induced rise in viscosity of the inventive aqueous gel composition, without a pharmaceutically active agent, with different poloxamer 407 contents.
Fig. 2a-: Temperature-induced rise in viscosity of the inventive aqueous gel composition with 15 % poloxamer 407 (a, left) compared to the same inventive aqueous gel composition containing 2% pharmaceutically active agent (API) which is (1S,2S,4R)-N-(3-(pentafluorosulfanyl)benzyl)bicyclo[2.2.1]heptane-2-carboxamide (ACOU085) (b, right).
Fig. 3a-b: Adhesion test: Pictures of gel formulation applied to a microscope object slide
Fig. 4. In vitro drug release test: time - release relations of the composition from Tab.1(new) containing 2% ACOU085 and 3% of ACOU085 (200µl dose) in triplicate.
Fig. 5. In vitro drug release test: time - release relations of the composition from Tab.1 (new) containing 2% of ACOU085 (300 µl Applied) and the reference formulation (ref) containing 3% ACOU085 (200 µl applied) in triplicate.
Fig. 6. In vitro drug release test: time - release relations of the composition from Tab.1 (new) containing 3% of ACOU085 (200 µl Applied) and the reference formulation (ref)containing 2% of ACOU085 (300 µl applied) in triplicate.
Fig. 7. Comparison of the composition from Tab.1 300 µl or 200 µl is applied.
Fig. 8: Comparison of the reference formulation 300 µl or 200 µl is applied.

### Viscosity test:

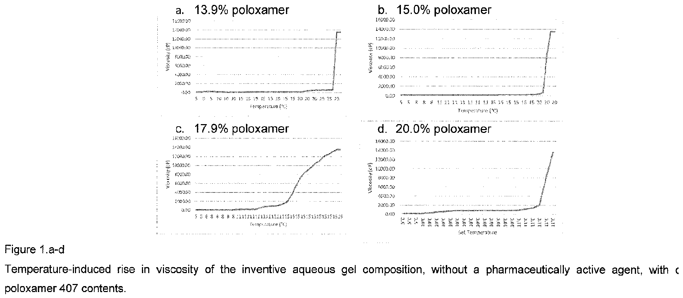

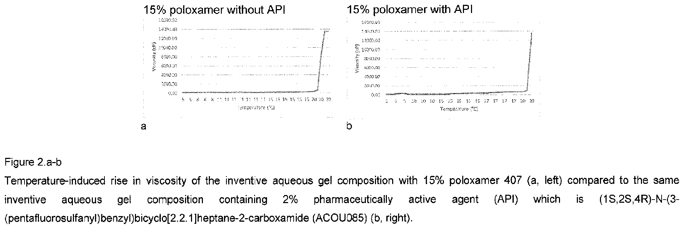

The temperature-induced rise in viscosity of the suspension was tested with the inventive aqueous gel composition (according to table 1), without a pharmaceutically active agent, containing 13.9%, 15%, 17.9% and 20% Poloxamer 407 (Fig. 1a-d and Fig. 2a).

Note: Maximal viscosity is identical for all formulations (due to the test method)

Fig. 1a-d shows that the turning point at which the viscosity rapidly increases upon a rise of temperature depends on the percentage of poloxamer. The composition with 15% poloxamer showed optimal viscosity characteristics as the viscosity rapidly increases at 20°C, which is favorable for application in medical practice.

Figure 2a-b shows that the pharmaceutically active agent (API) had no effect on the viscosity - temperature relation of the formulation.

### Adhesion test:

In an initial adhesion test the inventive aqueous gel composition (according to table 1) containing 14%, 15%, 17.9% and 20% poloxamer 407 was each applied on a finger. The composition containing 14% poloxamer 407 showed dripping of the composition from the finger. This dripping was not observed with 15%, 17.9% and 20%.

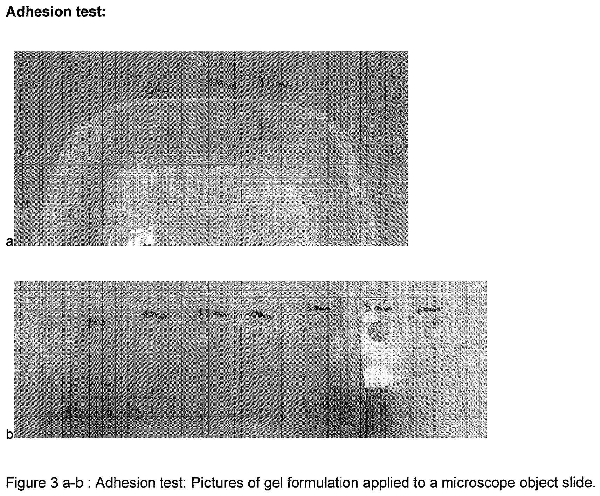

A final more precise adhesion test was performed with the inventive aqueous gel composition (according to table 1), without a pharmaceutically active agent, containing 14% poloxamer. 300 µl of this composition, was applied on microscopic object slides horizontally at room temperature. The slides were placed in an oven at 37°C and at several time points (30 sec, 1 min, 1.5 min, 2 min, 3 min, 5 min, 6min) the slides were turned vertically. Subsequent visual inspection showed that the compositions stayed in place at 37°C, no dripping was detected for all time points (see Fig. 3a-b).

As the inventive aqueous gel composition (according to table 1), without a pharmaceutically active agent, containing 14% poloxamer did not show any dripping in the final more precise adhesion test it can be assumed that also formulations with a higher poloxamer concentration will stay on the microscopic object slide because a higher poloxamer content will give a viscous composition at lower temperatures.

### Composition

**Table 1:**

| | Composition A | | Composition B | |
|---|---|---|---|---|
| Components | Amount in g | Percentage % | Amount in g | Percentage % |
| ACOU085 (API)** | 0.3 | 2.1 | 0.4 | 3.1 |
| Phosal 50PG | 2.0 | 13.8 | 2.0 | 13.8 |
| (phosphatidylcholine and propylene glycol 1: 1) | | | | |
| Water | 10.0 | 68.9 | 9.9 | 67.9 |
| Kolliphor P407 (poloxamer 407) | 2.2 | 15.2 | 2.2 | 15.2 |
| Total | 14.5 | 100 | 14.5 | 100 |

| | | | | |
|---|---|---|---|---|
| Composition of the inventive aqueous gel composition. Compositions used in the examples that deviate from the percentage or from the components mentioned in the table were regulated accordingly by filling up or omitting water. *: Exact percentages are not rounded numbers. **:+(1S,2S,4R)-N-(3-(pentafluorosulfanyl)benzyl)bicyclo[2.2.1]heptane-2-carboxamide (ACOU085) used for the formulation is GMP material with a particle diameter of: - D90: 32 µm. D90 means that 90% of the total particles are smaller than this diameter. - D50: 13 µm. D50 means that 50% of the total particles are smaller than this diameter. - D10: 3.5 µm. D10 means that 10% of the total particles are smaller than this diameter. | | | | |

### Composition for one vial of 550 mg:

**Table 1a:**

| Component | Reference to Quality Standards | Function | Unit Formula |
|---|---|---|---|
| ACOU085 (API) ** | In house monograph | Drug substance | 11.00 mg |
| Phosal 50 PG (phosphatidyl-choline and propylene glycol 1: 1) | Supplier's monograph | Surfactant, Alcohol | 75.90 mg |
| Kolliphor P407 (poloxamer 407) | Ph. Eur. | Surfactant | 83.60 mg |
| Water (for Injection) | Ph. Eur. | Solvent | 379.50 mg |
| Total Unit Weight | | | 550.00 mg |

| | | | |
|---|---|---|---|
| Composition of the inventive aqueous gel composition in mg. **+(1S,2S,4R)-N-(3-(pentafluorosulfanyl)benzyl)bicyclo[2.2.1]heptane-2-carboxamide | | | |

### Preparation

1. Mix Phosal 50PG (premixed phosphatidylcholine and propylene glycol, 1: 1) with (1S,2S,4R)-N-(3-(pentafluorosulfanyl)benzyl) bicyclo[2.2.1]heptane-2-carboxamide (ACOU085)
2. Add the water slowly while stirring with overhead stirrer (phase reversal from water in oil emulsion to oil in water emulsion)
3. Cool the mixture down on ice
4. Add kolliphor P407 (poloxamer 407) powder slowly during stirring with overhead stirrer
5. Keep the mixture cooled during the stirring and addition of poloxamer
6. Keep the mixture stirring for at least 1 hour until all kolliphor P407 is dissolved.

### Stability (stress) testing:

The prepared compositions from Table 1 have been stored at room temperature, for 6 months and have been visually tested for appearance. And showed no sign of degradation and/or precipitation.

### a) Analytical method

### In-vitro drug release test:

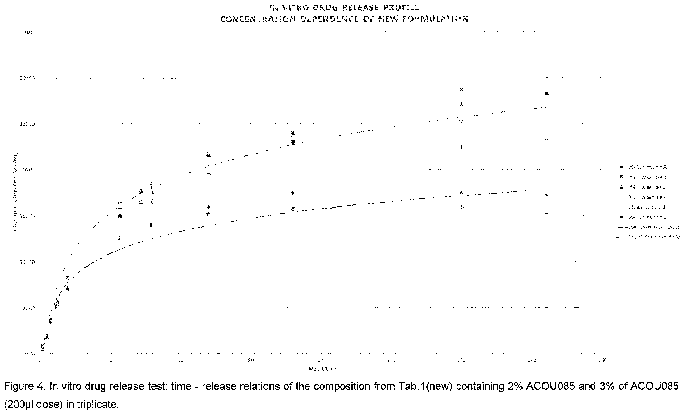

The optimal medium and membrane material have been determined:
i) Membrane:
   a. The polysulfone membrane was identified as optimal performing membrane as it showed excellent diffusion characteristics in the present tests (see Fig.4 to Fig. 7).
ii) Medium:
   a. Several media have been tested, and the optimal medium has been selected. PBS buffer pH 7.4 and 3% SDS was selected as optimal medium due to the properties and the good ACOU085 solubility and because air bubbles are not easily formed under the membrane (see Fig.4-7).

### b) Syringeability:

Syringeability of the inventive aqueous gel composition from Tab.1 with 1ml syringe with 21G, 25G needle is feasible.

### In vitro release data:

In vitro release data (Fig. 4 to Fig. 8 and Tab. 2 to Tab. 6) were amongst others performed with the Franz diffusion cell for permeation experiments. This device consists of two compartments with a fixed membrane (diffusion barrier) in between. The substance to be tested is applied directly to the top of the membrane and the concentration of the permeating compound can be detected on the opposite (acceptor) compartment.

If a reference composition was used, the reference composition is the following non-aqueous oleogel composition:
- ACOU085, present in an amount of 2 or 3 % by weight,
- castor oil, present in an amount of 92,5 or 91,5 % by weight,
- Labrafil^{®} (Oleoyl macrogolglycerides), present in an amount of 3,50 % by weight, and
- Silica, colloidal anhydrous, present in an amount of 2,00 % by weight.

**Table 2: In vitro drug release test: overview table of the result of the composition from Tab.1 containing 2% ACOU085 and 3% of ACOU085 (200 µl dose) in triplicate.**

| 2% ACOU085 with phosal 50PG and poloxamer | | | | | | | 3% ACOU085 with phosal 50 PG and poloxamer | | | | | | |
|---|---|---|---|---|---|---|---|---|---|---|---|---|---|
| | A | | B | | C | | | A | | B | | C | |
| Time hours | conc. Mg/mL | % of total amount of API | conc. Mg/mL | % of total amount of API | conc. Mg/mL | % of total amount of API | Time hours | conc. Mg/mL | % of total amount of API | conc. Mg/mL | % of total amount of API | conc. Mg/mL | % of total amount of API |
| 1 | 7.6 | 3.4 | 7.7 | 3.9 | 8.0 | 2.7 | 1 | 5.9 | 2.0 | 8.1 | 2.2 | 7.8 | 2.3 |
| 2 | 19.3 | 8.6 | 19.6 | 9.9 | 19.8 | 6.8 | 2 | 17.3 | 5.9 | 21.0 | 5.7 | 20.3 | 6.0 |
| 3 | 34.8 | 15.5 | 34.5 | 17.4 | 35.6 | 12.2 | 3 | 31.6 | 10.7 | 37.2 | 10.2 | 36.6 | 10.8 |
| 5 | 52.5 | 23.4 | 50.7 | 25.5 | 53.8 | 18.5 | 5 | 51.1 | 17.4 | 56.9 | 15.5 | 56.0 | 16.5 |
| 8 | 75.2 | 33.5 | 71.0 | 35.8 | 79.5 | 27.3 | 8 | 79.3 | 27.0 | 84.9 | 23.2 | 81.0 | 23.8 |
| 23 | 123.7 | 55.1 | 126.6 | 63.8 | 159.2 | 54.7 | 23 | 162.5 | 55.2 | 163.3 | 44.6 | 150.0 | 44.1 |
| 29 | 138.7 | 61.8 | 139.5 | 70.3 | 176.1 | 60.5 | 29 | 182.8 | 62.1 | 177.8 | 48.5 | 164.8 | 48.5 |
| 32 | 140.7 | 62.7 | 140.6 | 70.8 | 176.4 | 60.6 | 32 | 184.4 | 62.7 | 180.7 | 49.3 | 165.9 | 48.8 |
| 48 | 160.4 | 71.5 | 152.8 | 77.0 | 197.9 | 68.0 | 48 | 216.7 | 73.7 | 205.0 | 55.9 | 195.1 | 57.3 |
| 72 | 175.2 | 78.1 | 157.9 | 79.5 | 228.7 | 78.6 | 72 | 238.1 | 80.9 | 240.6 | 65.6 | 231.2 | 68.0 |
| 120 | 175.0 | 78.0 | 159.1 | 80.1 | 224.7 | 77.2 | 120 | 253.8 | 86.3 | 287.2 | 78.4 | 271.7 | 79.9 |
| 144 | 171.6 | 76.5 | 154.1 | 77.6 | 234.5 | 80.5 | 144 | 260.1 | 88.4 | 301.2 | 82.2 | 282.2 | 83.0 |

Figure 4 and Table 2 show that the formulation delivers a sustained, dose-dependent release across the test membrane into the medium. And over a period of 144 hrs the release was in range of 76.5-80.5% (2% ACOU085 formulation) to 82.2-88.4% (3% ACOU085 formulation) of the formulated pharmaceutical active agent.

**Table 3: In vitro drug release test: overview table of the results of reference formulation containing 3% ACOU085 (200 µl applied) in triplicate.**

| Reference product 3% | | | | | | | | | | | | |
|---|---|---|---|---|---|---|---|---|---|---|---|---|
| | Sample A | | | | Sample B | | | | Sample C | | | |
| Time hours | conc. µg/mL | % | conc. after cor. µg/mL | after cor. % | conc. µg/mL | % | conc. after cor. µg/mL | after cor. % | conc. µg/mL | % | conc. after cor. µg/mL | after cor. % |
| 1 | 32.2 | 10.7 | 32.2 | 10.7 | 29.4 | 9.4 | 29.4 | 9.4 | 30.0 | 10.0 | 30.0 | 10.0 |
| 2 | 53.7 | 17.8 | 54.0 | 17.9 | 48.3 | 15.4 | 48.6 | 15.5 | 49.6 | 16.5 | 49.9 | 16.6 |
| 4 | 81.4 | 27.0 | 82.3 | 27.3 | 73.8 | 23.5 | 74.6 | 23.8 | 72.0 | 23.9 | 72.8 | 24.2 |
| 8 | 109.3 | 36.3 | 111.0 | 36.9 | 104.7 | 33.4 | 106.3 | 33.9 | 101.4 | 33.6 | 102.9 | 34.1 |
| 12 | 125.2 | 41.6 | 128.0 | 42.5 | 119.0 | 38.0 | 121.6 | 38.8 | 121.7 | 40.4 | 124.2 | 41.2 |
| 24 | 151.3 | 50.3 | 155.3 | 51.6 | 142.7 | 45.5 | 146.4 | 46.7 | 146.9 | 48.7 | 150.6 | 50.0 |
| 32 | 157.9 | 52.4 | 163.4 | 54.3 | 147.5 | 47.1 | 152.7 | 48.7 | 153.7 | 51.0 | 158.9 | 52.7 |
| 48 | 164.8 | 54.7 | 171.9 | 57.1 | 151.0 | 48.2 | 157.7 | 50.3 | 161.0 | 53.4 | 167.8 | 55.7 |
| 120 | 184.6 | 61.3 | 193.4 | 64.2 | 158.4 | 50.6 | 166.6 | 53.2 | 171.3 | 56.8 | 179.6 | 59.6 |
| 144 | 175.2 | 58.2 | 185.8 | 61.7 | 158.2 | 50.5 | 168.0 | 53.6 | 173.1 | 57.4 | 183.1 | 60.8 |

**Table 4: In vitro drug release test: overview table of the results of the composition from Tab.1 containing 2% of ACOU085 (300 µl Applied)**

| Phosal 50PG formulation 2% ACOU085 and 15% poloxamer | | | | | | | | | | | | |
|---|---|---|---|---|---|---|---|---|---|---|---|---|
| | Sample A | | | | Sample B | | | | Sample C | | | |
| Time hours | conc. µg/mL | % | conc. after cor. µg/mL | after cor. % | conc. µg/mL | % | conc. after cor. µg/mL | after cor. % | conc. µg/mL | % | conc. after cor. µg/mL | after cor. % |
| 1 | 6.3 | 2.1 | 6.3 | 2.1 | 6.6 | 2.2 | 6.6 | 2.2 | 6.5 | 2.2 | 6.5 | 2.2 |
| 2 | 15.8 | 5.2 | 15.8 | 5.2 | 16.8 | 5.6 | 16.9 | 5.6 | 16.2 | 5.3 | 16.3 | 5.4 |
| 4 | 35.2 | 11.6 | 35.5 | 11.7 | 40.3 | 13.4 | 40.5 | 13.5 | 38.0 | 12.5 | 38.3 | 12.6 |
| 8 | 72.8 | 23.9 | 73.4 | 24.1 | 84.6 | 28.2 | 85.3 | 28.4 | 78.9 | 26.0 | 79.5 | 26.2 |
| 12 | 107.5 | 35.3 | 108.8 | 35.7 | 117.1 | 39.0 | 118.6 | 39.5 | 110.7 | 36.4 | 112.1 | 36.9 |
| 24 | 167.1 | 54.9 | 169.5 | 55.7 | 181.2 | 60.3 | 183.9 | 61.2 | 173.6 | 57.1 | 176.1 | 58.0 |
| 32 | 191.7 | 63.0 | 195.7 | 64.3 | 202.9 | 67.5 | 207.3 | 69.0 | 194.5 | 64.0 | 198.8 | 65.4 |
| 48 | 205.4 | 67.5 | 211.4 | 69.4 | 219.4 | 73.0 | 225.9 | 75.2 | 216.5 | 71.2 | 222.6 | 73.3 |
| 120 | 238.1 | 78.2 | 246.1 | 80.8 | 252.7 | 84.1 | 261.4 | 87.0 | 254.0 | 83.6 | 262.3 | 86.4 |
| 144 | 236.9 | 77.8 | 247.3 | 81.2 | 255.3 | 85.0 | 266.5 | 88.7 | 248.0 | 81.6 | 258.9 | 85.2 |

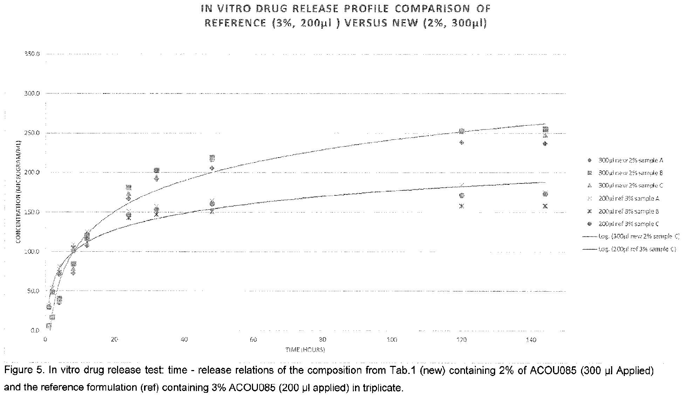

Figure 5, Table 3, and Table 4 show that both formulations deliver a sustained release across the test membrane into the medium. However, over a period of 144 hrs the release from the inventive aqueous gel composition leads to significantly higher drug concentrations in the receiving medium and a more complete release than from the reference formulation, despite overall identical amount of pharmaceutical active agent. In vitro release of the reference and inventive aqueous gel composition (new developed formulation) show similar curves. However, in the beginning (until 12hours) the reference shows slight faster release but, in the end, the inventive aqueous gel composition gives the highest concentration (Figure 5).

**Table 5: In vitro drug release test: overview table of the results of the reference formulation containing 2% of ACOU085 (300 µl applied) in triplicate.**

| Reference product 2% | | | | | | | | | | | | |
|---|---|---|---|---|---|---|---|---|---|---|---|---|
| | Sample A | | | | Sample B | | | | Sample C | | | |
| Time hours | conc. µg/mL | % | conc. after cor. µg/mL | after cor. % | conc. µg/mL | % | conc. after cor. µg/mL | after cor. % | conc. µg/mL | % | conc. after cor. µg/mL | after cor. % |
| 1 | 28.9 | 9.5 | 28.9 | 9.5 | 26.7 | 8.9 | 26.7 | 8.9 | 26.1 | 8.7 | 26.1 | 8.7 |
| 2 | 42.2 | 13.8 | 42.5 | 13.9 | 38.7 | 12.9 | 38.9 | 13.0 | 38.1 | 12.7 | 38.3 | 12.8 |
| 4 | 66.8 | 21.8 | 67.5 | 22.0 | 60.2 | 20.1 | 60.8 | 20.3 | 59.9 | 19.9 | 60.5 | 20.1 |
| 8 | 88.6 | 28.9 | 90.0 | 29.4 | 86.6 | 28.9 | 87.9 | 29.3 | 81.7 | 27.2 | 82.9 | 27.6 |
| 12 | 102.3 | 33.4 | 104.6 | 34.2 | 98.2 | 32.8 | 100.3 | 33.5 | 95.1 | 31.6 | 97.2 | 32.3 |
| 24 | 121.4 | 39.6 | 124.7 | 40.7 | 118.8 | 39.6 | 121.9 | 40.7 | 116.2 | 38.7 | 119.2 | 39.7 |
| 32 | 129.7 | 42.4 | 134.2 | 43.8 | 126.0 | 42.0 | 130.3 | 43.5 | 128.1 | 42.6 | 132.3 | 44.0 |
| 48 | 139.4 | 45.5 | 145.2 | 47.4 | 126.2 | 42.1 | 131.7 | 44.0 | 132.7 | 44.1 | 138.1 | 46.0 |
| 120 | 141.8 | 46.3 | 149.0 | 48.6 | 141.8 | 47.3 | 148.6 | 49.6 | 144.3 | 48.0 | 151.0 | 50.3 |
| 144 | 145.3 | 47.4 | 153.9 | 50.3 | 148.6 | 49.6 | 156.9 | 52.3 | 150.0 | 49.9 | 158.2 | 52.7 |

**Table 6: In vitro drug release test: time - release relations of the composition from Tab.1 containing 3% of ACOU085 (200 µl Applied) in triplicate.**

| Phosal50PG formulation 3% ACOU085 and 15% poloxamer | | | | | | | | | | | | |
|---|---|---|---|---|---|---|---|---|---|---|---|---|
| | Sample A | | | | Sample B | | | | Sample C | | | |
| Time hours | conc. µg/mL | % | conc. after cor. µg/mL | after cor. % | conc. µg/mL | % | conc. after cor. µg/mL | after cor. % | conc. µg/mL | % | conc. after cor. µg/mL | after cor. % |
| 1 | 9.3 | 3.0 | 9.3 | 3.0 | 9.2 | 3.0 | 9.2 | 3.0 | 9.1 | 3.0 | 9.1 | 3.0 |
| 2 | 17.5 | 5.6 | 17.6 | 5.6 | 17.8 | 5.8 | 17.9 | 5.8 | 17.5 | 5.7 | 17.6 | 5.7 |
| 4 | 38.9 | 12.4 | 39.2 | 12.5 | 41.0 | 13.4 | 41.3 | 13.5 | 39.0 | 12.7 | 39.3 | 12.8 |
| 8 | 84.8 | 27.1 | 85.4 | 27.3 | 77.6 | 25.3 | 78.3 | 25.5 | 81.9 | 26.7 | 82.5 | 26.9 |
| 12 | 115.1 | 36.8 | 116.6 | 37.3 | 105.1 | 34.3 | 106.6 | 34.7 | 113.0 | 36.8 | 114.4 | 37.3 |
| 24 | 171.0 | 54.7 | 173.7 | 55.5 | 162.1 | 52.8 | 164.6 | 53.6 | 177.6 | 57.9 | 180.2 | 58.8 |
| 32 | 187.2 | 59.8 | 191.6 | 61.2 | 184.9 | 60.2 | 189.0 | 61.6 | 207.0 | 67.5 | 211.4 | 68.9 |
| 48 | 234.6 | 75.0 | 240.8 | 77.0 | 223.8 | 72.9 | 229.8 | 74.9 | 251.8 | 82.1 | 258.3 | 84.2 |
| 120 | 255.3 | 81.6 | 263.8 | 84.3 | 230.0 | 74.9 | 238.2 | 77.6 | 274.7 | 89.5 | 283.6 | 92.5 |
| 144 | 266.7 | 85.2 | 277.8 | 88.8 | 262.4 | 85.5 | 272.9 | 88.9 | 282.0 | 91.9 | 293.7 | 95.7 |

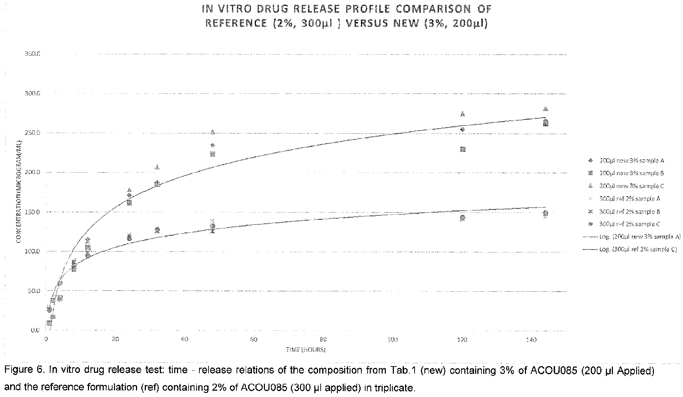

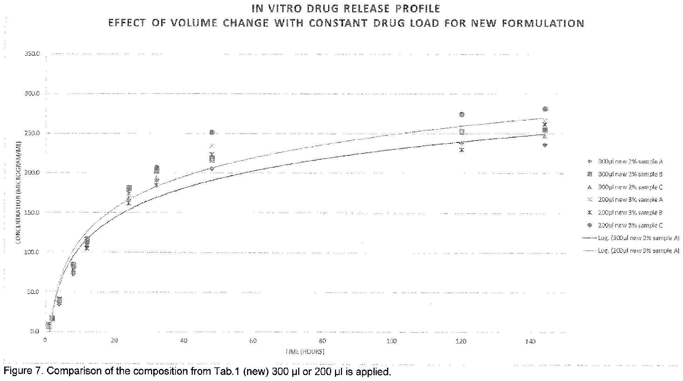

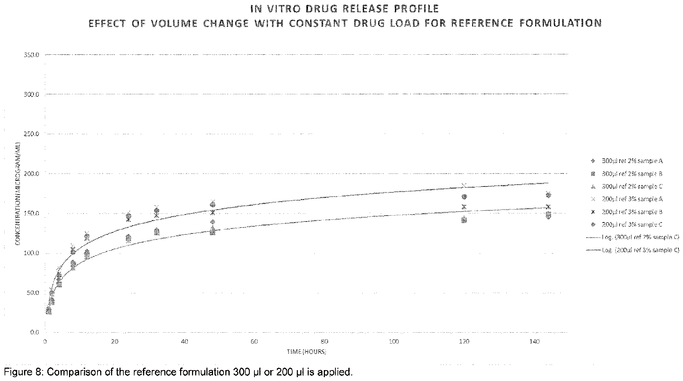

Figure 6 and Table 5 and Table 6 show that both formulations deliver a sustained release across the test membrane into the recipient medium. However, over a period of 144 hrs the release from the inventive aqueous gel composition leads to significantly higher drug concentrations in the receiving medium and a more complete release than from the reference formulation, despite overall identical amount of pharmaceutical active agent. This advantage was not influenced by changing respectively formulation volume/ pharmaceutical active agent content (300 µl /2% ACOU085 vs 200 µl /3% ACOU085) between the inventive aqueous gel composition and reference oleogel formulation. Figure 7 and Figure 8 show that the higher achieved pharmaceutical active agent concentration in the receiving medium and more complete release of the loaded pharmaceutical active agent was specific to the inventive aqueous gel composition, compared to the reference composition, and for each formulation was independent of the combination of volume/ pharmaceutical active agent concentration for constant drug load.

### Influence of a solubility agent to water ratio on pharmaceutically active agents that have relatively poor water solubility properties

Solubility enhancing agents are investigated to improve solubility of pharmaceutically active agents that have relatively poor water solubility properties in water.

As a pharmaceutically active agent that has relatively poor solubility properties in water (1S,2S,4R)-N-(3-(pentafluorosulfanyl)benzyl) bicyclo[2.2.1]heptane-2-carboxamide (ACOU085) was used.

To start, six mixtures are prepared in order to see if an emulsion with ACOU085 in water is a feasible approach. As soon as a stable emulsion is formed, the mixture can be further stabilized with other compounds, in particular poloxamer 407, added to the water phase. Poloxamer 407 introduces thermo-reversible properties to the mixture.

| **Ingredients** | **Mixture 1** | **Mixture 2** | **Mixture 3** | **Mixture 4** |
|---|---|---|---|---|
| Premix Phosal 50PG and ACOU085 | 1 drop (0.033mL) | 1 mL (30drops) | 1 drop (0.033mL) | 1 drop (0.033mL |
| Water | 10 mL | 10 mL | 10 mL | 9 mL |
| Polysorbate 80 | | | | 0.25 g |
| Cremophor RH40 | | | 1 drop (40°C) | |

**Table 7: Overview of the tested mixtures. The premix of Phosal 50PG and ACOU085 included 3 g Phosal 50PG, 0.6 g ACOU085.**

| **Ingredients** | **Mixture 5** | **Mixture 6** |
|---|---|---|
| Phosal 50PG | 1 mL | 1 mL |
| Water | 20 mL | 5 mL |

Mixture 1: Composition 1 drop of premix Phosal 50PG + ACOU085 + 10 mL of milliQ water.

Preparation method: 1. Pipette 1 drop Phosal 50PG with ACOU085 in 10 mL of water. 2. Formation of flakes but not stable 3. Sonication for 10 min formation of white to light blue haze, typical for micro/nano-emulsions.

After addition of the premix to water, flake formation occurred. These flakes were removed by sonication for 10 min. After sonication a white to light blue haze was formed which is typical for micro/nano-emulsions.

Mixture 2: Composition 1 mL of premix Phosal 50PG + ACOU085 + 10 mL of milliQ water.

Preparation method: 1. Pipette 1 mL of Phosal 50PG with ACOU085 in 10 mL of water. 2. Formation of large flakes but not stable 3. Sonication for 10 min formation of white milky emulsion 4. Precipitation after a few days but not stable.

After the addition of the premix to the water, flake formation occurs. After 10 min of sonication a milky emulsion is formed but precipitation occurred after a few days. Thus, the mixture is not stable. Additionally, a microscopic picture of mixture 2 shows large ACOU085 particles, which are not incorporated in the formed emulsion or precipitated shortly after preparation.

Mixture 3: Composition 1 drop of premix Phosal 50PG + ACOU085 + 10 mL of milliQ water + 1 drop of Cremophor RH40 at 40 °C.

Preparation method: 1. Water with Cremophor at 40 °C. 2. Pipette 1 drop of Phosal + ACOU085 into the mixture during stirring. 3. Formation of flakes but further stirring let the flakes dissolve.

After addition of the premix to water and cremophor mixture, flakes occur but after stirring these flakes dissolve. The mixture is opaque and remains stable. No visible ACOU085 particles are present in the microscopic pictures.

Mixture 4: Composition 1 g of premix Phosal 50PG + ACOU085 + 0.25 g polysorbate 80 + 9 g of milliQ water.

Preparation method: 1. Mix Phosal 50PG + ACOU085 with the 0.25 g Polysorbate 80. 2. Add the water slow under stirring. 3. Phase reversal, first formation of w/o emulsion, further addition of water o/w (oil in water) emulsion. The mixture was prepared using phase-reversal technique. First a water in oil emulsion is formed and after further addition of water, an oil in water emulsion is formed. The formulation is a milky emulsion that remains stable at first, but after 10 days, it precipitates. In the microscopic pictures, a few particles of ACOU085 are visible.

Mixture 5: Composition 1 mL Phosal 50PG+ 20 mL MilliQ water.

Preparation method: 1. Stir the water. 2. Slowly add Phosal 50PG to the water phase. 3. Stir after each drop until homogeneous. Mixture 5 was prepared without ACOU085 to obtain a first idea if it is feasible to prepare a stable emulsion with Phosal 50PG and water. 4. Mixing of water and slow addition of Phosal 50 PG.

### Mixture 6: Composition 1 mL of Phosal 50PG + 5 g of milliQ water

### Preparation method: 1. Stir the Phosal 50 PG. 2. Add the water slowly. 3. phase reversal w/o emulsion to o/w emulsion, product is liquid

The attempt of preparing a stable emulsion in mixture 5 was not successful. Therefore, another approach is used to prepare a stable emulsion. This mixture was also prepared without ACOU085 to obtain a first idea if the phase reversal technique might work. First Phosal 50PG is added and water is added slowly during stirring of the Phosal 50PG. The mixture becomes a viscous water in oil emulsion and after addition of more water, an oil in water emulsion is formed. The mixture is a milky emulsion that remains stable (see Figure 10). In the microscopic picture a typical image of an emulsion is shown with small homogeneously distributed droplets.

### Conclusion:

Out of these 6 mixtures it can be concluded that the emulsion of mixture 6 showed the best stability and has the possibility to emulsify a sufficient amount of ACOU085 in order to create a workable end formulation. This mixture is prepared using Phase-reversal technique, which result in small homogeneously distributed droplets. This mixture is used as a basis to further develop the formulation with thermos-reversible properties. Apparently a Phosal:water ratio of 1:5, preferably 1:1 to 1:10, more preferably 1:1,5 to 1:3 showed the best stability.

### Mixture 6 with ACOU085 and a compound with thermo-reversible properties

Three formulations were prepared according to the Phosal 50G and water ratio as described in mixture 6 in order to see if a stable and homogeneous suspension with thermo-reversible properties was possible.

**Table 8: Formulation with thermo-reversible properties**

| Components | Formulation 8 | Formulation 9 | Formulation 10 |
|---|---|---|---|
| Phosal 50PG | 2.0 g | | 2.0 g |
| ACOU085 | 0.4 g (Batch CC5875.0-15) | 0.4 g (Batch CC5875.0-15) | 0.3 g (Batch CC-5876.0-01) |
| Water | 10.0 g | | 10.0 g |
| Labrafill | | 2.0 g | |
| Kolliphor P407 | 3.0 g | | 3.0 g |
| Pluronic 20% (f127) | | 15.0 g | |

These Formulations lead to stable formulations with the desired thermo-reversible properties. However, formulations with Labrafill showed impaired thermo-reversible properties compared to formulations with Kolliphor P407 (also known as Poloxamer 407).

### In vivo release data

Eighteen female Sprague Dawley rats entered a pharmacokinetic study, to determine the distribution of a 6% thermo-reversible ACOU085 formulation at a dose of 1.2 mg/animal after unilateral transtympanic administration in peripheral plasma, inner ear, perilymph, brain and CSF. 4, 24, 48, 96, 168 and 336 h after dosing n=3 rats out of every group were euthanized with carbon dioxide, peripheral and final blood samples and brain were taken and inner ears were dissected to analyze ACOU085 concentrations. Body weight was assessed on the day 1-4, on day 7 and day 14. Animals were checked for any clinical signs associated with toxicity of the test substance.

No visible signs of pain, behavioral changes or adverse reactions were noted after the treatment with the sponsors test substance and all animals survived their scheduled study period. Only slight defensive reactions such as head scratching were observed directly after injection. It is believed that this reaction was due to the fluid remaining in the middle ear after injection. This lack of obvious macroscopically detectable reaction suggests that no adverse effects leading to animal response (pain, discomfort, loss of balance) were caused by the sponsors test substances when transtympanically applied in the middle ear of female Sprague Dawley rats at a dose of 1.2 mg/animal.

**Table 9: Uptake of ACOU085 into the plasma**

| **Animal ID** | **ACOU085 plasma concentration (nnmol/L)** | | | | | |
|---|---|---|---|---|---|---|
| | **4 h** | **24 h** | **48 h** | **96 h** | **168 h** | **336 h** |
| 1 | 9 | 19 | 12 | 48 | 30 | 9 |
| 2 | 6 | 113 | 18 | 26 | 33 | 2 0 |
| 3 | 12 | 2 | 16 | 33 | 15 | |

**Table 10: Uptake of ACOU085 into the brain**

| **Animal ID** | **ACOU085 brain concentration (nnmol/L)** | | | | | |
|---|---|---|---|---|---|---|
| | **4 h** | **24 h** | **48 h** | **96 h** | **168 h** | **336 h** |
| 1 | 29 | 54 | 38 | 94 | 115 | 12 |
| 2 | 14 | 116 | 53 | 52 | 81 | 10 |
| 3 | 39 | 9 | 76 | 68 | 49 | 1 |

Uptake of ACOU085 into the brain and into the plasma was low in comparison to the inner ear tissue and perilymph as indicated by the low concentrations measured in these organs. Indeed, the maximal average concentration measured was only 45 nmol/L in plasma and 82 nmol/L in brain.

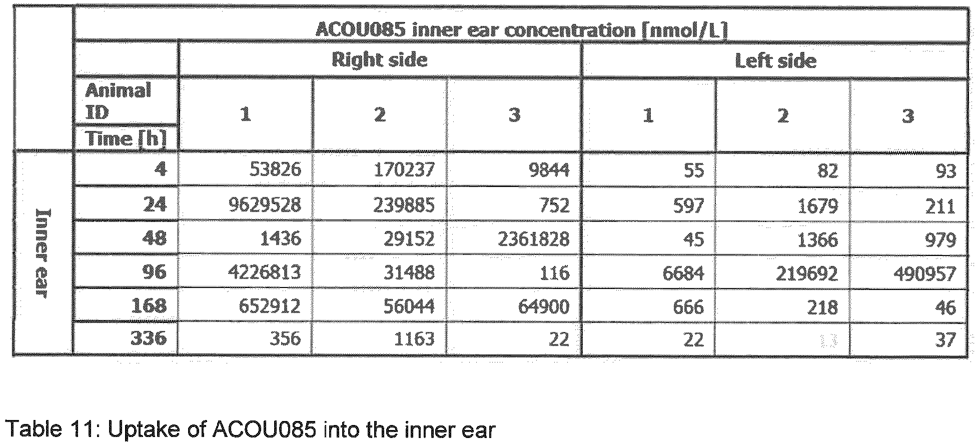

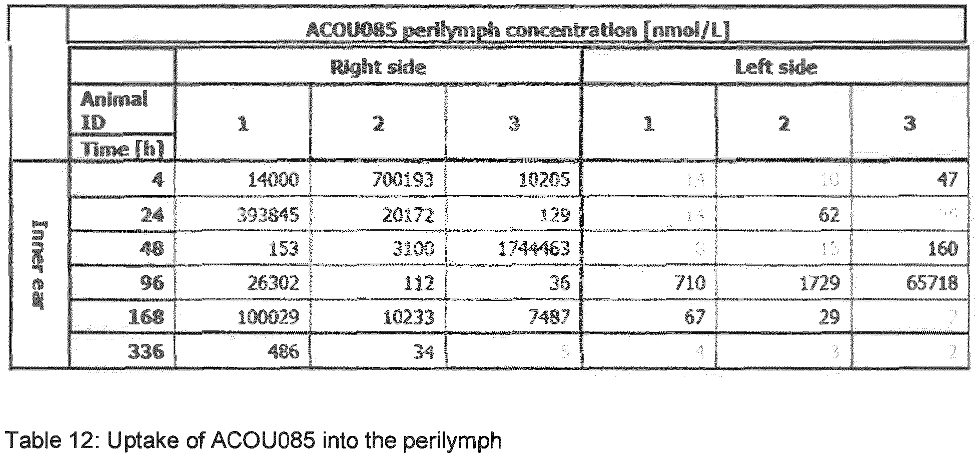

ACOU085 was still present at detectable levels in the plasma after 7 days (26 nmol/L), but concentrations decreased steadily reaching 4 nmol/L 14 days after administration. In the brain, ACOU085 concentration increased consistently after treatment, (Figure 3) reaching a peak at 96 h after injection. After 96 h ACOU085 concentrations steadily decreased until, similar to the plasma, they were almost undetectable 14 days after administration (8 nmol/L).

### Conclusion:

Plasma pharmacokinetics (PK) and distribution of a pharmaceutically active agent, using 6% ACOU085 in the inventive thermoreversible, aqueous gel composition administered unilaterally transtympanically in female Sprague Dawley rats showed an improved tolerability and release profile compared to other formulations leading to no or only a few side effects and a sustained release of the pharmaceutically active agent.

## Claims

1. Aqueous gel composition comprising
- at least one first surfactant in an amount from 10 % to 30 % by weight, based on the total weight of the composition,
- at least one second surfactant in an amount from 5 % to 20 % by weight, based on the total weight of the composition,
- at least one alcohol in an amount from 5 % to 20 % by weight, based on the total weight of the composition,
- and water in an amount of at least 30 % by weight, based on the total weight of the composition, and
- at least one pharmaceutically active agent having a mean particle diameter from 0,01 µm to 100 µm,
wherein
- the at least one first surfactant is a poloxamer,
- the at least one second surfactant is a phospholipid,
- the at least one alcohol is propylene glycol and
- the at least one pharmaceutically active agent has the formula I: 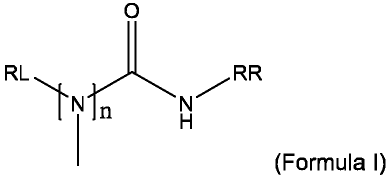 wherein
- n = 1,
- RL is a substituent selected from the group consisting of substituted cyclopentathienyl groups, wherein said substituted cyclopentathienyl groups are substituted with at least one F-atom or Cl-atom, and of unsubstituted or substituted indanyl groups, wherein said substituted indanyl groups are substituted with at least one F-atom or Cl-atom, and
- RR is a substituted phenyl group, wherein said substituted phenyl group comprises at least one substituent selected from F, SF₅, CF₃, and OCF₃
- or the at least one pharmaceutically active agent has the formula II: 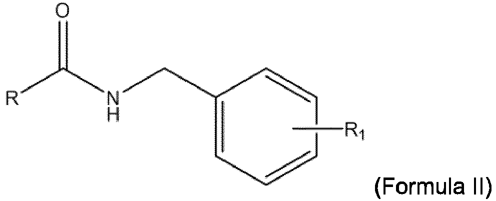 wherein
- R is an unsubstituted cycloalkyl group, in particular bicycloalkyl group,
an unsubstituted or substituted phenyl group, or an unsubstituted or substituted thienyl group, wherein said substituted thienyl group or phenyl group is substituted with at least one halogen, preferably at least one F-atom or at least one Cl-atom, and
- R₁ is F, SF₅, CF₃ or OCF₃.

2. Aqueous gel composition according to claim 1, wherein the viscosity of the composition at a temperature of 25°C is from 1400 mPas to 2300 mPas, preferably from 1600 mPas to 2000 mPas.

3. Aqueous gel composition according to claim 1 or 2, wherein the at least one phospholipid is selected from the group consisting of phosphatidylcholine, cholesterol, phosphatidylethanolamine, phosphatidylserine and mixtures of at least two of the afore-said phospholipids.

4. Aqueous gel composition according to anyone of the preceding claims, wherein the at least one first surfactant is present in an amount from 10 % to 25 % by weight, preferably from 12 % to 22 % by weight, more preferably from 14 % to 16 % by weight, based on the total weight of the composition.

5. Aqueous gel composition according to anyone of the preceding claims, wherein the at least one second surfactant is present in an amount from 6 % to 20 % by weight, preferably from 6 % to 15 % by weight, more preferably from 6 % to 10 % by weight, based on the total weight of the composition.

6. Aqueous gel composition according to anyone of the preceding claims, wherein the at least one alcohol is present in an amount from 6 % to 20 % by weight, preferably from 6 % to 15 % by weight, more preferably from 6 % to 10 % by weight, based on the total weight of the composition.

7. Aqueous gel composition according to anyone of the preceding claims, wherein:
- poloxamer is present in an amount from 14 % to 16 % by weight, based on the total weight of the composition,
- phospholipid, preferably a natural phosphatidylcholine is present in an amount from 6 % to 10 % by weight, based on the total weight of the composition, and
- propylene glycol is present in an amount from 6 % to 10 % by weight, based on the total weight of the composition.

8. Aqueous gel composition according to anyone of the preceding claims, wherein the at least one pharmaceutically active agent is present in an amount from 0,05 % to 10 % by weight, preferably from 0,1 % to 10 % by weight, more preferably from 2 % to 10 % by weight, based on the total weight of the composition.

9. Aqueous gel composition according to anyone of the preceding claims, wherein the at least one pharmaceutically active agent has a mean particle diameter from 5 µm to 80 µm, preferably from 20 µm to 50 µm.

10. Aqueous gel composition according to anyone of the preceding claims, wherein the at least one pharmaceutically active agent has the formular I and is selected from the group consisting of
(S)-3-(3,4-difluorophenyl)-1-(2-chloro-5,6-dihydro-4H-cyclopenta[b]thiophen-6-yl)-1-methylurea,
(S)-3-(3-pentafluorosulfanylphenyl)-1-(2-chloro-5,6-dihydro-4H-cyclopenta[b]thiophen-6-yl)-1-methylurea,
(S)-3-(3,4-difluorophenyl)-1-(2,3-dihydro-1H-inden-1yl)-1-methylurea,
(S)-3-(3,4-difluorophenyl)-1-(5-chloro-2,3-dihydro-1H-inden-1yl)-1-methylurea,
(S)-3-(3,4-difluorophenyl)-1-(5-fluoro-2,3-dihydro-1H-inden-1yl)-1-methylurea, and
(S)-1-(5-chloro-2,3-dihydro-1-H-inden-1-yl)-1-methyl-3-(3-pentafluorosulfanylphenyl) urea,
and mixtures of at least two of the afore-said pharmaceutically active agents.

11. Aqueous gel composition according to anyone of the preceding claims, wherein the at least one pharmaceutically active agent has the formular II and is selected from the group consisting of
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide,
(1S,2S,4R)-N-(3-(trifluoromethoxy)benzyl)bicyclo[2.2.1]heptane-2-carboxamide,
p-Chloro-N-(4-trifluoromethoxy)benzyl)benzamide,
p-Chloro-N-(4-(pentafluorosulfanyl)benzyl)benzamide,
p-Fluoro-N-(4-trifluoromethoxy)benzyl)benzamide,
p-Fluoro-N-(4-(pentafluorosulfanyl)benzyl)benzamide,
p-Chloro-N-(4-(trifluoromethyl)benzyl)benzamide,
p-Fluoro-N-(4-(trifluoromethyl)benzyl)benzamide,
and mixtures of at least two of the afore-said pharmaceutically active agents.

12. Pharmaceutical composition or medicament comprising an aqueous gel composition according to anyone of the preceding claims.

13. Aqueous gel composition according to anyone of claims 1 to 11, for the use in the prevention or treatment of inner ear diseases or a pharmaceutical composition according to claim 12 for use in the prevention or treatment of inner ear diseases.

## Patentansprüche

1. Wässrige Gelzusammensetzung, umfassend
- mindestens ein erstes Tensid in einer Menge von 10 bis 30 Gew.-%, bezogen auf das Gesamtgewicht der Zusammensetzung,
- mindestens ein zweites Tensid in einer Menge von 5 bis 20 Gew.-%, bezogen auf das Gesamtgewicht der Zusammensetzung,
- mindestens einen Alkohol in einer Menge von 5 bis 20 Gew.-%, bezogen auf das Gesamtgewicht der Zusammensetzung,
- und Wasser in einer Menge von mindestens 30 Gew.-%, bezogen auf das Gesamtgewicht der Zusammensetzung, und
- mindestens einen pharmazeutischen Wirkstoff mit einem mittleren Teilchendurchmesser von 0,01 µm bis 100 µm,
wobei
- es sich bei dem mindestens einen ersten Tensid um ein Poloxamer handelt,
- es sich bei dem mindestens einen zweiten Tensid um ein Phospholipid handelt,
- es sich bei dem mindestens einen Alkohol um Propylenglykol handelt und
- der mindestens eine pharmazeutische Wirkstoff die Formel I aufweist: 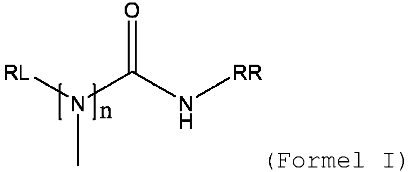 wobei
- n = 1,
- RL für einen Substituenten steht, der aus der Gruppe bestehend aus substituierten Cyclopentathienylgruppen, wobei die substituierten Cyclopentathienylgruppen durch mindestens ein F-Atom oder Cl-Atom substituiert sind, und aus unsubstituierten oder substituierten Indanylgruppen, wobei die substituierten Indanylgruppen durch mindestens ein F-Atom oder Cl-Atom substituiert sind, ausgewählt ist, und
- RR für eine substituierte Phenylgruppe steht, wobei die substituierte Phenylgruppe mindestens einen Substituenten, der aus F, SF₅, CF₃ und OCF₃ ausgewählt ist, umfasst,
- oder der mindestens eine pharmazeutische Wirkstoff die Formel II aufweist: 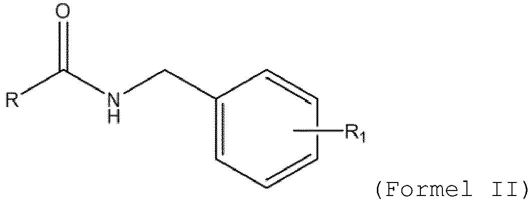 wobei
- R für eine unsubstituierte Cycloalkylgruppe, insbesondere Bicycloalkylgruppe,
eine unsubstituierte oder substituierte Phenylgruppe oder eine unsubstituierte oder substituierte Thienylgruppe steht, wobei die substituierte Thienylgruppe oder Phenylgruppe durch mindestens ein Halogen, vorzugsweise mindestens ein F-Atom oder mindestens ein Cl-Atom, substituiert ist, und
- R₁ für F, SF₅, CF₃ oder OCF₃ steht.

2. Wässrige Gelzusammensetzung nach Anspruch 1, wobei die Viskosität der Zusammensetzung bei einer Temperatur von 25 °C 1400 mPas bis 2300 mPas, vorzugsweise 1600 mPas bis 2000 mPas, beträgt.

3. Wässrige Gelzusammensetzung nach Anspruch 1 oder 2, wobei das mindestens eine Phospholipid aus der Gruppe bestehend aus Phosphatidylcholin, Cholesterin, Phosphatidylethanolamin, Phosphatidylserin und Mischungen von mindestens zwei der zuvor genannten Phospholipide ausgewählt ist.

4. Wässrige Gelzusammensetzung nach einem der vorhergehenden Ansprüche, wobei das mindestens eine erste Tensid in einer Menge von 10 bis 25 Gew.-%, vorzugsweise von 12 bis 22 Gew.-%, weiter bevorzugt von 14 bis 16 Gew.-%, bezogen auf das Gesamtgewicht der Zusammensetzung, vorliegt.

5. Wässrige Gelzusammensetzung nach einem der vorhergehenden Ansprüche, wobei das mindestens eine zweite Tensid in einer Menge von 6 bis 20 Gew.-%, vorzugsweise von 6 bis 15 Gew.-%, weiter bevorzugt von 6 bis 10 Gew.-%, bezogen auf das Gesamtgewicht der Zusammensetzung, vorliegt.

6. Wässrige Gelzusammensetzung nach einem der vorhergehenden Ansprüche, wobei der mindestens eine Alkohol in einer Menge von 6 bis 20 Gew.-%, vorzugsweise von 6 bis 15 Gew.-%, weiter bevorzugt von 6 bis 10 Gew.-%, bezogen auf das Gesamtgewicht der Zusammensetzung, vorliegt.

7. Wässrige Gelzusammensetzung nach einem der vorhergehenden Ansprüche, wobei:
- Poloxamer in einer Menge von 14 bis 16 Gew.-%, bezogen auf das Gesamtgewicht der Zusammensetzung, vorliegt,
- Phospholipid, vorzugsweise ein natürliches Phosphatidylcholin, in einer Menge von 6 bis 10 Gew.-%, bezogen auf das Gesamtgewicht der Zusammensetzung, vorliegt und
- Propylenglykol in einer Menge von 6 bis 10 Gew.-%, bezogen auf das Gesamtgewicht der Zusammensetzung, vorliegt.

8. Wässrige Gelzusammensetzung nach einem der vorhergehenden Ansprüche, wobei der mindestens eine pharmazeutische Wirkstoff in einer Menge von 0,05 bis 10 Gew.-%, vorzugsweise von 0,1 bis 10 Gew.-%, weiter bevorzugt von 2 bis 10 Gew.-%, bezogen auf das Gesamtgewicht der Zusammensetzung, vorliegt.

9. Wässrige Gelzusammensetzung nach einem der vorhergehenden Ansprüche, wobei der mindestens eine pharmazeutische Wirkstoff einen mittleren Teilchendurchmesser von 5 µm bis 80 µm, vorzugsweise von 20 µm bis 50 µm, aufweist.

10. Wässrige Gelzusammensetzung nach einem der vorhergehenden Ansprüche, wobei der mindestens eine pharmazeutische Wirkstoff die Formel I aufweist und ausgewählt ist aus einer Gruppe bestehend aus
(S)-3-(3,4-Difluorphenyl)-1-(2-chlor-5,6-dihydro-4H-cyclopenta[b]thiophen-6-yl)-1-methylharnstoff,
(S)-3-(3-Pentafluorsulfanylphenyl)-1-(2-chlor-5,6-dihydro-4H-cyclopenta[b]thiophen-6-yl)-1-methylharnstoff,
(S)-3-(3,4-Difluorphenyl)-1-(2,3-dihydro-1H-inden-1-yl)-1-methylharnstoff,
(S)-3-(3,4-Difluorphenyl)-1-(5-chlor-2,3-dihydro-1H-inden-1-yl)-1-methylharnstoff,
(S)-3-(3,4-Difluorphenyl)-1-(5-fluor-2,3-dihydro-1H-inden-1-yl)-1-methylharnstoff, und
(S)-1-(5-Chlor-2,3-dihydro-1-H-inden-1-yl)-1-methyl-3-(3-pentafluorsulfanylphenyl)harnstoff
und Mischungen von mindestens zwei der zuvor genannten pharmazeutischen Wirkstoffe.

11. Wässrige Gelzusammensetzung nach einem der vorhergehenden Ansprüche, wobei der mindestens eine pharmazeutische Wirkstoff die Formel II aufweist und ausgewählt ist aus einer Gruppe bestehend aus
(1R,2R,4S)-rel-N-(3-(Pentafluor-λ6-sulfanyl)benzyl)bicyclo[2.2.1]heptan-2-carboxamid,
(1S,2S,4R)-N-(3-(Pentafluor-λ6-sulfanyl)benzyl)bicyclo[2.2.1]heptan-2-carboxamid,
(1R,2R,4S)-rel-N-(3-(Trifluormethyl)benzyl)bicyclo[2.2.1]heptan-2-carboxamid,
(1R,2R,4S)-rel-N-(3-(Trifluormethoxy)benzyl)bicyclo[2.2.1]heptan-2-carboxamid,
(1S,2S,4R)-N-(3-(Trifluormethyl)benzyl)bicyclo[2.2.1]heptan-2-carboxamid,
(1S,2S,4R)-N-(3-(Trifluormethoxy)benzyl)bicyclo[2.2.1]-heptan-2-carboxamid,
p-Chlor-N-(4-trifluormethoxy)benzyl)benzamid,
p-Chlor-N-(4-(pentafluorsulfanyl)benzyl)benzamid,
p-Fluor-N-(4-trifluormethoxy)benzyl)benzamid,
p-Fluor-N-(4-(pentafluorsulfanyl)benzyl)benzamid,
p-Chlor-N-(4-(trifluormethyl)benzyl)benzamid,
p-Fluor-N-(4-(trifluormethyl)benzyl)benzamid
und Mischungen von mindestens zwei der zuvor genannten pharmazeutischen Wirkstoffe.

12. Pharmazeutische Zusammensetzung oder Medikament, umfassend eine wässrige Gelzusammensetzung nach einem der vorhergehenden Ansprüche.

13. Wässrige Gelzusammensetzung nach einem der Ansprüche 1 bis 11 zur Verwendung bei der Prävention oder Behandlung von Innenohrerkrankungen oder pharmazeutische Zusammensetzung nach Anspruch 12 zur Verwendung bei der Prävention oder Behandlung von Innenohrerkrankungen.

## Revendications

1. Composition de gel aqueux comprenant
- au moins un premier tensioactif en une quantité de 10 % à 30 % en poids, par rapport au poids total de la composition,
- au moins un deuxième agent tensioactif en une quantité de 5 % à 20 % en poids, par rapport au poids total de la composition,
- au moins un alcool en une quantité de 5 % à 20 % en poids, par rapport au poids total de la composition,
- et de l'eau en une quantité d'au moins 30 % en poids, par rapport au poids total de la composition, et
- au moins un agent pharmaceutiquement actif ayant un diamètre moyen de particule de 0,01 µm à 100 µm,
dans laquelle
- l'au moins un premier tensioactif est un poloxamère,
- l'au moins un deuxième tensioactif est un phospholipide,
- l'au moins un alcool est le propylène glycol et
- l'au moins un agent pharmaceutiquement actif a la formule I : 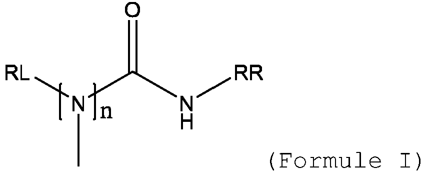 dans laquelle
- n = 1,
- RL est un substituant choisi dans le groupe constitué par des groupes cyclopentathiényle substitués, dans laquelle lesdits groupes cyclopentathiényle substitués sont substitués par au moins un atome de F ou atome de Cl, et parmi des groupes indanyle non substitués ou substitués, dans laquelle lesdits groupes indanyle substitués sont substitués par au moins un atome de F ou atome de Cl, et
- RR est un groupe phényle substitué, dans laquelle ledit groupe phényle substitué comprend au moins un substituant choisi parmi F, SF₅, CF₃, et OCF₃
- ou l'au moins un agent pharmaceutiquement actif a la formule II : 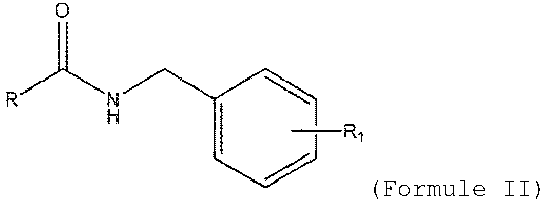 dans laquelle
- R est un groupe cycloalkyle non substitué, en particulier un groupe bicycloalkyle,
un groupe phényle non substitué ou substitué, ou un groupe thiényle non substitué ou substitué, dans laquelle ledit groupe thiényle ou groupe phényle substitué est substitué par au moins un halogène, de préférence au moins un atome de F ou au moins un atome de Cl, et
- R₁ est F, SF₅, CF₃ ou OCF₃.

2. Composition de gel aqueux selon la revendication 1, dans laquelle la viscosité de la composition à une température de 25 °C est de 1 400 mPas à 2 300 mPas, de préférence de 1 600 mPas à 2 000 mPas.

3. Composition de gel aqueux selon la revendication 1 ou 2, dans laquelle l'au moins un phospholipide est choisi dans le groupe constitué par la phosphatidylcholine, le cholestérol, la phosphatidyléthanolamine, la phosphatidylsérine et des mélanges d'au moins deux desdits phospholipides précédents.

4. Composition de gel aqueux selon l'une quelconque des revendications précédentes, dans laquelle l'au moins un premier tensioactif est présent en une quantité de 10 % à 25 % en poids, de préférence de 12 % à 22 % en poids, mieux encore de 14 % à 16 % en poids, par rapport au poids total de la composition.

5. Composition de gel aqueux selon l'une quelconque des revendications précédentes, dans laquelle l'au moins un deuxième tensioactif est présent en une quantité de 6 % à 20 % en poids, de préférence de 6 % à 15 % en poids, mieux encore de 6 % à 10 % en poids, par rapport au poids total de la composition.

6. Composition de gel aqueux selon l'une quelconque des revendications précédentes, dans laquelle l'au moins un alcool est présent en une quantité de 6 % à 20 % en poids, de préférence de 6 % à 15 % en poids, mieux encore de 6 % à 10 % en poids, par rapport au poids total de la composition.

7. Composition de gel aqueux selon l'une quelconque des revendications précédentes, dans laquelle :
- le poloxamère est présent en une quantité de 14 % à 16 % en poids, par rapport au poids total de la composition,
- le phospholipide, de préférence une phosphatidylcholine naturelle, est présent en une quantité de 6 % à 10 % en poids, par rapport au poids total de la composition, et
- le propylène glycol est présent en une quantité de 6 % à 10 % en poids, par rapport au poids total de la composition,

8. Composition de gel aqueux selon l'une quelconque des revendications précédentes, dans laquelle l'au moins un agent pharmaceutiquement actif est présent en une quantité de 0,05 % à 10 % en poids, de préférence de 0,1 % à 10 % en poids, mieux encore de 2 % à 10 % en poids, par rapport au poids total de la composition.

9. Composition de gel aqueux selon l'une quelconque des revendications précédentes, dans laquelle l'au moins un agent pharmaceutiquement actif a un diamètre moyen de particule de 5 à 80 µm, de préférence de 20 µm à 50 µm.

10. Composition de gel aqueux selon l'une quelconque des revendications précédentes, dans laquelle l'au moins un agent pharmaceutiquement actif a la formule I et est choisi dans le groupe constitué par
(S)-3-(3,4-difluorophényl)-1-(2-chloro-5,6-dihydro-4H-cyclopenta[b]thiophén-6-yl)-1-méthylurée,
(S)-3-(3-pentafluorosulfanylphényl)-1-(2-chloro-5,6-dihydro-4H-cyclopenta[b]thiophén-6-yl)-1-méthylurée,
(S)-3-(3,4-difluorophényl)-1-(2,3-dihydro-1H-indén-1-yl)-1-méthylurée,
(S)-3-(3,4-difluorophényl)-1-(5-chloro-2,3-dihydro-1H-indén-1-yl)-1-méthylurée,
(S)-3-(3,4-difluorophényl)-1-(5-fluoro-2,3-dihydro-1H-indén-1-yl)-1-méthylurée, et
(S)-1-(5-chloro-2,3-dihydro-1-H-indén-1-yl)-1-méthyl-3-(3-pentafluorosulfanyl-phényl)urée,
et des mélanges d'au moins deux desdits agents pharmaceutiquement actifs précédents.

11. Composition de gel aqueux selon l'une quelconque des revendications précédentes, dans laquelle l'au moins un agent pharmaceutiquement actif a la formule II et est choisi dans le groupe constitué par
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-A6-sulfanyl)benzyl)bicyclo[2.2.1]heptane-2-carboxamide,
(1R,2R,4S)-rel-N-(3-(trifluorméthyl)benzyl)bicyclo[2.2.1]heptane-2-carboxamide,
(1R,2R,4S)-rel-N-(3-(trifluorométhoxy)benzyl)bicyclo[2.2.1]heptane-2-carboxamide,
(1S,2S,4R)-N-(3-(trifluorométhyl)benzyl)bicyclo[2.2.1]heptane-2-carboxamide,
(1S,2S,4R)-N-(3-(trifluorométhoxy)benzyl)bicyclo[2.2.1]heptane-2-carboxamide,
p-Chloro-N-(4-trifluorométhoxy)benzyl)benzamide,
p-Chloro-N-(4-(pentafluorosulfanyl)benzyl)benzamide,
p-Fluoro-N-(4-trifluorométhoxy)benzyl)benzamide,
p-Fluoro-N-(4-(pentafluorosulfanyl)benzyl)benzamide,
p-Chloro-N-(4-trifluorométhyl)benzyl)benzamide,
p-Fluoro-N-(4-(trifluorométhyl)benzyl)benzamide,
et des mélanges d'au moins deux desdits agents pharmaceutiquement actifs précédents.

12. Composition pharmaceutique ou médicament comprenant une composition de gel aqueux selon l'une quelconque des revendications précédentes.

13. Composition de gel aqueux selon l'une quelconque des revendications 1 à 11, pour l'utilisation dans la prévention ou le traitement de maladies de l'oreille interne ou composition pharmaceutique selon la revendication 12 pour utilisation dans la prévention ou le traitement de maladies de l'oreille interne.
